# EUROPÄISCHE PATENTSCHRIFT

(11) **EP 2 410 972 B1**
(45) Veröffentlichungstag und Bekanntmachung des Hinweises auf die Patenterteilung: **12.10.2016**
(21) Anmeldenummer: 10707266.2
(22) Anmeldetag: 02.03.2010
(51) Int. Cl.: A61K 8/03, A61K 8/22, A61K 8/891, A61Q 5/10, A61Q 5/08, A61K 8/58

(54) **ZWEIPHASEN-ENTWICKLER**
TWO-PHASE DEVELOPER
RÉVÉLATEUR À DEUX PHASES

(30) Priorität: 27.03.2009 DE 102009001937
(43) Veröffentlichungstag der Anmeldung: 01.02.2012
(73) Patentinhaber: Henkel AG & Co. KGaA, 40589 Düsseldorf (DE)
(72) Erfinder: RATH, Susanne, 20359 Hamburg (DE); KLEEN, Astrid, 20457 Hamburg (DE)
(86) Internationale Anmeldenummer: PCT/EP2010/052602
(87) Internationale Veröffentlichungsnummer: WO 2010/108763

(56) Entgegenhaltungen:
- WO-A1-2008/132816
- FR-A1- 2 910 309
- US-A1- 2003 211 953
- US-A1- 2008 172 807
- US-A1- 2009 041 699
- US-B1- 6 238 653

## Beschreibung

Gegenstand der vorliegenden Anmeldung ist eine kosmetisches Mittel für die Behandlung keratinischer Fasern, insbesondere menschlicher Haare, welche sich durch zwei voneinander getrennte Phasen auszeichnet, wobei eine der Phasen eine wässrige Phase und die andere Phase eine hydrophobe Phase darstellt, wobei letztere mindestens ein Siliconöl, ausgewählt aus Dimethiconen und Cyclomethiconen, enthält. Diese Mittel enthalten zumindest ein chemisches Oxidationsmittel und weiterhin mindestens ein Siliconöl. In ihrer Eigenschaft als oxidierende Zubereitungen werden diese Mittel als Entwicklerzubereitung für Oxidationsfärbungen oder Aufhellmittel eingesetzt. Ein weiterer Gegenstand der vorliegenden Erfindung ist daher ein Verfahren zur Farbveränderung keratinsicher Fasern, bei dem die erfindungsgemäßen Mittel zur Farbveränderung auf keratinische Fasern aufgebracht werden. Die Veränderung von Form und Farbe der Haare stellt einen wichtigen Bereich der modernen Kosmetik dar. Dadurch kann das Erscheinungsbild der Haare sowohl aktuellen Modeströmungen als auch den individuellen Wünschen der einzelnen Person angepasst werden. Diese Mittel sollen neben der gewünschten Färbe und Formleistung möglichst minimale Schädigungen auf dem Haar hervorrufen und vorzugsweise sogar zusätzliche Pflegeeigenschaften besitzen.

Zur Bereitstellung farbverändernder kosmetischer Mittel, insbesondere für die Haut oder keratinhaltige Fasern wie beispielsweise menschliche Haare, kennt der Fachmann je nach Anforderungen an die Färbung diverse Färbesysteme. Für permanente, intensive Färbungen mit entsprechenden Echtheitseigenschaften werden sogenannte Oxidationsfärbemittel verwendet. Solche Färbemittel enthalten üblicherweise Oxidationsfarbstoffvorprodukte, sogenannte Entwicklerkomponenten und Kupplerkomponenten, die unter dem Einfluss von Oxidationsmitteln oder von Luftsauerstoff untereinander die eigentlichen Farbstoffe ausbilden. Die Oxidationsfärbemittel zeichnen sich durch hervorragende, lang anhaltende Färbeergebnisse aus. Für temporäre Färbungen werden üblicherweise Färbe- oder Tönungsmittel verwendet, die als färbende Komponente sogenannte Direktzieher enthalten.

Neben der Färbung ist das Aufhellen der eigenen Haarfarbe bzw. das Blondieren der ganz spezielle Wunsch vieler Verbraucher, da eine blonde Haarfarbe als attraktiv und in modischer Hinsicht erstrebenswert betrachtet wird. Sollen Substrate aufgehellt oder gar gebleicht werden, werden die das Substrat färbenden Farbstoffe meist oxidativ unter Einsatz von entsprechenden Oxidationsmitteln, wie beispielsweise Wasserstoffperoxid, entfärbt.

Insbesondere oxidative Haarbehandlungsmittel sind trotz ihrer vorteilhaften Färbe- und/oder Aufhelleigenschaften für den Anwender mit Nachteilen behaftet. Einerseits führt der Einsatz der Oxidationsmittel zu Schädigungen in der Haarstruktur und auf der Haaroberfläche. Das Haar wird brüchig, seine Elastizität lässt nach und die Kämmbarkeit nimmt ab. Diese Schädigung nimmt mit der Anwendungsdauer zu. Andererseits benötigen oxidative Färbemittel in der Regel einen alkalischen pH-Wert zur Ausfärbung, insbesondere zwischen pH 9,0 und pH 10,5. Die mit dem basischen pH-Wert verbundene Spreizung der äußeren Schuppenschicht des Haares führt jedoch zu einem unangenehmen Oberflächenempfinden der Haare und damit zu einer verschlechterten Kämmbarkeit im Nass- und Trockenzustand. Dadurch besteht für den Verbraucher eine gesteigerte Notwendigkeit, zusätzliche Nachbehandlungsmittel wie Konditioniermittel einsetzen. Nicht zuletzt wird die Haarstruktur jedoch auch durch äußere Umwelteinflüsse in Mitleidenschaft gezogen. Hierzu sind mechanische und thermische Einwirkungen, wie Kämmen und Fönen, zu zählen. Ebenso tragen Wettereinflüsse, wie Wind, Regen und UV-Strahlung im Sonnenlicht, und zusätzliche äußere Belastungen, wie beispielsweise chloriertes Schwimmbadwasser oder Schweiß, zur Schädigung der Haarstruktur und der Haaroberfläche bei. Um den Pflegezustand der Fasern zu verbessern, ist es seit langem üblich, die Fasern im Anschluss an die farbverändernde Behandlung einer speziellen Nachbehandlung zu unterziehen. Dabei werden, üblicherweise in Form einer Spülung, die Haare mit speziellen Wirkstoffen, beispielsweise quaternären Ammoniumsalzen oder speziellen Polymeren, behandelt. Durch diese Behandlung werden je nach Formulierung Kämmbarkeit, Halt und Fülle der Haare verbessert und die Splißrate verringert.

US20030211953A1 lehrt Haarbehandlungsmittel, die "funktionalisierte" Silicone enthalten. Die "funktionalisierten" Silicone erlauben eine länger haltbare Pflegewirkung auf insbesondere durch Haarfärbung und Haarbleiche geschädigten Haaren. Die organo-modifizierten Silicone sind bevorzugt in ein wasserunlösliches Organosiloxan-Harz eingebettet. Bevorzugte Produktform ist eine Emulsion, insbesondere eine Silicon-in-Wasser-Emulsion, in der die Silicon-Tröpfchen durch Emulgatoren stabilisiert werden. FR2910309A1 betrifft zweikomponentige, oxidative Färbemittel, die eine Siliconzubereitung aus X und Y enthalten, die in Gegenwart von Peroxiden eine Hydrosilylierungsreaktion miteinander eingehen. Erschwerend ist hierbei, dass viele der üblichen Pflege-und Wirkstoffe zur Verringerung der Haarschädigung unter den oxidativen Bedingungen eines Haarbehandlungsmittels nur unzureichend stabil sind.

Es besteht daher weiterhin ein Bedarf an pflegenden Zubereitungen für die farbverändernde Behandlung von Fasern. Aufgabe der vorliegenden Erfindung ist es daher, ein farbveränderndes Mittel zur Verfügung zu stellen, durch welches die oben genannten Nachteile gebräuchlicher farbverändernden Mittel herabgesenkt werden. Insbesondere Schutz vor oxidativen Schädigungen der Haarstruktur und der Haaroberfläche soll durch die farbverändernden Mittel erzielt werden. Besonders wünschenswert sind pflegende Eigenschaften der Mittel, so dass der Anwender auf den Einsatz zusätzlicher Konditionier- und Nachbehandlungsmittel verzichten kann. Dazu sollen die Mittel auch geeignet sein, oxidativ unzureichend stabile Pflege- und Wirkstoffe zu stabilisieren und so in der oxidativen Haarbehandlung einsetzbar machen. Weiterhin ist für den Anwender wünschenswert, wenn die Mittel zusätzlich zur objektiven Pflegeleistung eine visuelle Verdeutlichung der Pflege offenbaren.

In nicht vorhersehbarer Weise konnte nun gefunden werden, dass durch spezielle zweiphasige kosmetische, oxidative Mittel für den Einsatz in farbverändernden Mitteln für keratinische Fasern, insbesondere menschliche Haare, die Haarschädigung minimiert werden kann oder sogar eine Haarpflege erzielt werden.

Ein erster Gegenstand der vorliegenden Erfindung ist daher ein kosmetisches Mittel für die Behandlung keratinischer Fasern, das mindestens zwei voneinander getrennte Phasen umfasst, wobei die erste Phase (I) eine wässrige Phase darstellt, die mindestens ein chemisches Oxidationsmittel enthält, und wobei die zweite Phase (II) eine hydrophobe Phase darstellt, die mindestens ein Siliconöl enthält, dadurch gekennzeichnet, dass die beiden Phasen in zwei Schichten übereinander vorliegen,
dass das Mittel nichtionische, anionische, zwitterionische und/oder amphotere Tenside und/oder Emulgatoren in einem Gesamtgewicht von weniger als 5 Gew.-%, bezogen auf das Gesamtgewicht des Mittels, enthält,
dass das Mittel als hydrophobe Phase (II) mindestens ein Siliconöl, ausgewählt aus der Gruppe, die gebildet wird aus Dimethiconen und Cyclomethiconen, enthält und
dass das Mittel zusätzlich mindestens eine überwiegend im Siliconöl lösliche Pflegekomponente, ausgewählt aus öllöslichen Pflegestoffen, öllöslichen Vitaminen und Triglyceriden, enthält.

Unter keratinhaltigen bzw. keratinischen Fasern werden erfindungsgemäß Pelze, Wolle, Federn und insbesondere menschliche Haare verstanden. Obwohl die erfindungsgemäße Verwendung in erster Linie zum Färben und/oder Aufhellen von keratinhaltigen Fasern geeignet sind, steht prinzipiell einer Verwendung auch auf anderen Gebieten nichts entgegen.

Entscheidendes Merkmal des erfindungsgemäßen Mittels ist dabei die Zweiphasigkeit, wobei die beiden Phasen nicht miteinander mischbar sind. Die beiden Phasen liegen in zwei Schichten übereinander vor.

In dem erfindungsgemäßen Mittel liegt die Phase (I) bevorzugt zu mindestens gleichen Gewichtsanteil wie die Phase (II) vor. Bevorzugt liegt Phase (I) im Überschuss vor. Bevorzugt besitzt das Gewichtsverhältnis von Phase (I) zu Phase (II) einen Wert von 99 zu 1 bis 50 zu 50, bevorzugt von 98 zu 2 bis 80 zu 20, besonders bevorzugt von 95 zu 5 bis 90 zu 10. Gemäß der vorliegenden Erfindung besitzt die erste Phase (I) einen wässrigen oder wässrig-alkoholischen Träger. Unter wässrig-alkoholischen Trägern sind im Sinne der vorliegenden Erfindung wasserhaltige Zusammensetzungen, enthaltend 3 bis 70 Gew.-% eines C₁-C₄-Alkohols, bezogen auf das Gesamtgewicht der Anwendungsmischung, insbesondere Ethanol bzw. Isopropanol, zu verstehen. Ein wässriger Träger enthält im Sinne der Erfindung mindestens 30 Gew.-%, insbesondere mindestens 50 Gew.-% Wasser, bezogen auf das Gesamtgewicht der Anwendungsmischung.

Weiterhin enthält die erste Phase (I) mindestens ein chemisches Oxidationsmittel. Der Begriff "chemisches Oxidationsmittel" soll dabei verdeutlichen, dass es sich dabei um ein extra zugesetztes Oxidationsmittel handelt und nicht etwa um ein in der Umgebung vorliegendes Oxidationsmittel, wie beispielsweise Luftsauerstoff. Als erfindungsgemäßes Oxidationsmittel wird bevorzugt Wasserstoffperoxid verwendet. Wasserstoffperoxid wird dabei entweder als vorzugsweise wässrige Lösung oder in Form einer festen Anlagerungsverbindung von Wasserstoffperoxid an anorganische oder organische Verbindungen, wie beispielsweise Natriumperborat, Natriumpercarbonat, Magnesiumpercarbonat, Natriumpercarbamid, Polyvinylpyrrolidinon·n H₂O₂ (n ist eine positive ganze Zahl größer 0), Harnstoffperoxid und Melaminperoxid, eingesetzt. Erfindungsgemäß besonders bevorzugte wässrige Phasen (I) enthalten wässrige Wasserstoffperoxid-Lösungen. Die Konzentration einer Wasserstoffperoxid-Lösung wird einerseits von den gesetzlichen Vorgaben und andererseits von dem gewünschten Effekt bestimmt. Vorzugsweise werden als wässrige Phase 3 Gew.-%ige bis 12 Gew.-%ige Lösungen in Wasser verwendet.

Die erfindungsgemäßen Zubereitungen enthalten mit besonderem Vorzug Wasserstoffperoxid. Hier sind erfindungsgemäße Mittel zur Farbveränderung keratinischer Fasern besonders bevorzugt, die 0,5 bis 18 Gew.-%, vorzugsweise 1 bis 15 Gew.-%, besonders bevorzugt 2,5 bis 12 Gew.-% und insbesondere 3 bis 9 Gew.-% Wasserstoffperoxid (berechnet als 100%iges H₂O₂) enthalten. Gemäß der vorliegenden Erfindung ist die zweite Phase (II) hydrophober Natur. Die erfindungsgemäße hydrophobe Phase (II) ist mit der wässrigen Phase (I), enthaltend das Oxidationsmittel, nicht mischbar. Hydrophobe Phasen, auch lipophile Phasen genannt, enthalten Fettkörper, die üblicherweise unpolare organische Verbindungen wie Kohlenwasserstoffverbindungen, langkettige Triglyceride, Ester oder Ether sowie perhalogenierte Verbindungen. Eine weitere Substanzklasse, die dazu geeignet ist, hydrophobe Phasen auszubilden sind Silicone, insbesondere Siliconöle. Die hydrophobe Phase (II) der vorliegenden Verbindung zeichnet sich dadurch aus, dass sie mindestens ein Siliconöl, ausgewählt aus der Gruppe, die gebildet wird aus Dimethiconen und Cyclomethiconen, enthält. Der Begriff "Siliconöl" bezeichnet dabei bei Raumtemperatur und unter Normaldruck flüssige Silicone. Silicone verfügen dabei über sogenannte Siloxan-Bindungen, was zu der gleichwertigen Bezeichnung Siloxan führte. Erfindungsgemäße geeignete Siliconöle sind solche, die keine oder nur eine sehr geringe Wasserlöslichkeit besitzen.

Besonders bevorzugte erfindungsgemäße kosmetische Mittel sind dadurch gekennzeichnet, dass sie mindestens ein Silicon der Formel (Si-1)

(CH₃)₃Si-[OSi(CH₃)₂]ₓ-OSi(CH₃)₃ (Si-1),

enthalten, in der x für eine Zahl von 0 bis 100, vorzugsweise von 0 bis 50, weiter bevorzugt von 0 bis 20 und insbesondere 0 bis 10, steht.

Die erfindungsgemäß eingesetzten, bevorzugten kosmetischen Mittel enthalten ein Silicon der vorstehenden Formel (Si-1). Diese Silicone werden nach der INCI-Nomenklatur als Dimethicone bezeichnet. Es werden im Rahmen der vorliegenden Erfindung als Silicon der Formel (Si-1) vorzugsweise die Verbindungen:
(CH₃)₃Si-OSi(CH₃)₃ ; (CH₃)₃Si-OSi(CH₃)₂-OSi(CH₃)₃ ; (CH₃)₃Si-[OSi(CH₃)₂]₂-OSi(CH₃)₃ ; (CH₃)₃Si-[OSi(CH₃)₂]₃-OSi(CH₃)₃ ; (CH₃)₃Si-[OSi(CH₃)₂]₄-OSi(CH₃)₃ ; (CH₃)₃Si-[OSi(CH₃)₂]₅-OSi(CH₃)₃ ; (CH₃)₃Si-[O-Si(CH₃)₂]₆-OSi(CH₃)₃ ; (CH₃)₃Si-[OSi(CH₃)₂]₇-OSi(CH₃)₃ ; (CH₃)₃Si-[OSi(CH₃)₂]₈-OSi(CH₃)₃ ; (CH₃)₃Si-[OSi(CH₃)₂]₉-OSi(CH₃)₃ ; (CH₃)₃Si-[OSi(CH₃)₂]₁₀-OSi(CH₃)₃ ; (CH₃)₃Si-[OSi(CH₃)₂]₁₁-OSi(CH₃)₃ ; (CH₃)₃Si-[OSi(CH₃)₂]₁₂-OSi(CH₃)₃ ; (CH₃)₃Si-[OSi(CH₃)₂]₁₃-OSi(CH₃)₃ ; (CH₃)₃Si-[OSi(CH₃)₂]₁₄-OSi(CH₃)₃ ; (CH₃)₃Si-[OSi(CH₃)₂]₁₅-OSi(CH₃)₃ ; (CH₃)₃Si-[OSi(CH₃)₂]₁₆-OSi(CH₃)₃ ; (CH₃)₃Si-[OSi(CH₃)₂]₁₇-OSi(CH₃)₃ ; (CH₃)₃Si-[OSi(CH₃)₂]₁₈-O-Si(CH₃)₃ ; (CH₃)₃Si-[OSi(CH₃)₂Si]₁₉-O(CH₃)₃ ; (CH₃)₃Si-[OSi(CH₃)₂]₂₀-OSi(CH₃)₃ eingesetzt, wobei (CH₃)₃Si-OSi(CH₃)₃, (CH₃)₃Si-OSi(CH₃)₂-OSi(CH₃)₃ und/oder (CH₃)₃Si-[OSi(CH₃)₂]₂-OSi(CH₃)₃ besonders bevorzugt sind.

Selbstverständlich können auch Mischungen der o.g. Silicone in den bevorzugten erfindungsgemäßen Mitteln enthalten sein.

Bevorzugte erfindungsgemäß einsetzbare Silicone weisen bei 20°C Viskositäten von 0,2 bis 2 mm²s⁻¹ auf, wobei Silicone mit Viskositäten von 0,5 bis 1 mm²s⁻¹ besonders bevorzugt sind.

Auch die nach INCI als Cyclomethicone bezeichneten cyclischen Dimethicone sind erfindungsgemäß mit Vorzug einsetzbar. Hier sind erfindungsgemäße kosmetische oder dermatologische Zubereitungen bevorzugt, die mindestens ein Silicon der Formel (Si-2), 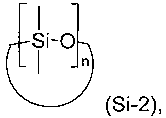 enthalten, in der x für eine Zahl von 0 bis 200, vorzugsweise von 0 bis 10, weiter bevorzugt von 0 bis 7 und insbesondere 0, 1, 2, 3, 4, 5 oder 6, steht.

Bevorzugt enthält das erfindungsgemäße Mittel das Siliconöl zu 0,1 bis 50 Gew.-%, bevorzugt zu 0,5 bis 30 Gew.-%, und insbesondere zu 1 bis 15 Gew.-%, jeweils bezogen auf das Gesamtgewicht des anwendungsbereiten Mittels.

Um die Trennung von hydrophiler Phase (I) und hydrophober Phase (II) zu verbessern, ist es erfindungsgemäß bevorzugt, wenn das Mittel nur einen geringen Anteil an grenzflächenaktiven Substanzen enthält. Als grenzflächenaktive Substanzen im Sinne der Erfindung gelten dabei Emulgatoren und Tenside. Grenzflächenaktive Substanzen zeichnen sich durch hydrophobe und hydrophile Strukturmerkmale aus und ermöglichen so eine Durchmischung der Phasen unter Ausbildung von Micellen und stabilen Emulsionen. Da die vorliegende Erfindung explizit keine Emulsionen umfasst, sondern vielmehr zwei getrennt voneinander vorliegende Phasen enthält, hat es sich als erfindungsgemäß besonders vorteilhaft herausgestellt, dass das Mittel nichtionische, anionische, zwitterionische und/oder amphotere Tenside und/oder Emulgatoren in einem Gesamtgewicht von weniger als 5 Gew.-%, bevorzugt weniger als 1 Gew.-%, jeweils bezogen auf das Gesamtgewicht des anwendungsbereiten Mittels, enthält. Besonders vorteilhaft sind Mittel, die frei sind von grenzflächenaktiven Substanzen.

Anionische Tenside im Sinne der Erfindung sind alle für die Verwendung am menschlichen Körper geeigneten anionischen oberflächenaktiven Stoffe. Diese sind gekennzeichnet durch eine wasserlöslich machende, anionische Gruppe wie beispielsweise eine Carboxylat-, Sulfat-, Sulfonat- oder Phosphat-Gruppe und eine lipophile Alkylgruppe mit etwa 8 bis 30 C-Atomen. Zusätzlich können im Molekül Glykol- oder Polyglykolether-Gruppen, Ester-, Ether- und Amidgruppen sowie Hydroxylgruppen enthalten sein. Beispiele für solche anionischen Tenside sind, jeweils in Form der Natrium-, Kalium- und Ammonium- sowie der Mono, Di- und Trialkanolammoniumsalze mit 2 bis 4 C-Atomen in der Alkanolgruppe, lineare und verzweigte Fettsäuren mit 8 bis 30 C-Atomen (Seifen); Ethercarbonsäuren, insbesondere der Formel RO(CH₂CH₂O)ₓCH₂COOH, in der R eine lineare Alkylgruppe mit 8 bis 30 C-Atomen und x = 0 oder 1 bis 16 ist; Acylsarcoside; Acyltauride; Acylisethionate; Sulfobernsteinsäuremono- und -dialkylester sowie Sulfobernsteinsäuremono-alkylpolyoxyethylester; lineare Alkansulfonate; lineare α-Olefinsulfonate; Sulfonate ungesättigter Fettsäuren; α-Sulfofettsäuremethylester von Fettsäuren; Alkylsulfate und Alkylethersulfate, insbesondere der Formel RO(CH₂CH₂O)ₓSO₃H, in der R für eine lineare Alkylgruppe mit 8 bis 30 C-Atomen und x für 0 oder eine Zahl von 1 bis 12 steht; Gemische oberflächenaktiver Hydroxysulfonate; sulfatierte Hydroxyalkylpolyethylen- und/oder Hydroxyalkylenpropylenglykolether; Ester der Weinsäure und Zitronensäure mit Alkoholen; Alkyl- und/oder Alkenyletherphosphate der Formel RO(C₂H₄O)ₓP(=O)(OH)(OR'), worin R für einen aliphatischen, gegebenenfalls ungesättigten Kohlenwasserstoffrest mit 8 bis 30 Kohlenstoffatomen, R' für Wasserstoff, einen Rest (CH₂CH₂O)_{y}R und x und y unabhängig voneinander für eine Zahl von 1 bis 10 steht; sulfatierte Fettsäurealkylenglykolester der Formel RC(O)O(alkO)ₙSO₃H, in der R für einen linearen oder verzweigten, aliphatischen, gesättigten und/oder ungesättigten Alkylrest mit 6 bis 22 C-Atomen, alk für CH₂CH₂, CHCH₃CH₂ und/oder CH₂CHCH₃ und n für eine Zahl von 0,5 bis 5 steht; sowie Monoglyceridsulfate und Monoglyceridethersulfate.

Als zwitterionische Tenside werden solche oberflächenaktiven Verbindungen bezeichnet, die im Molekül mindestens eine quartäre Ammoniumgruppe und mindestens eine Carboxylat-, Sulfonat-oder Sulfat-Gruppe tragen. Beispiele solcher zwitterionischen Tenside sind die sogenannten Betaine wie die N-Alkyl-N,N-dimethylammonium-glycinate, beispielsweise das Kokosalkyl-dimethylammoniumglycinat, N-Acyl-aminopropyl-N,N-dimethylammoniumglycinate, beispielsweise das Kokosacylaminopropyl-dimethylammoniumglycinat, und 2-Alkyl-3-carboxymethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Unter amphoteren Tensiden werden solche oberflächenaktiven Verbindungen verstanden, die außer einer C₈-C₂₄-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Übliche amphotere Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 24 C-Atomen in der Alkylgruppe. Beispielhafte amphotere Tenside sind N-Kokosalkylaminopropionat, Kokosacylaminoethylaminopropionat und C₁₂-C₁₈-Acylsarcosin.

Nichtionische Tenside und Emulgatoren enthalten als hydrophile Gruppe z. B. eine Polyolgruppe, eine Polyalkylenglykolethergruppe oder eine Kombination aus Polyol- und Polyglykolethergruppe. Solche Verbindungen sind beispielsweise Anlagerungsprodukte von 1 bis 50 Mol Ethylenoxid und/oder 0 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe; mit einem Methyl- oder C₂-C₆-Alkylrest endgruppenverschlossene Anlagerungsprodukte von 1 bis 50 Mol Ethylenoxid und/oder 0 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe, wie beispielsweise die unter den Verkaufsbezeichnungen Dehydol LS, Dehydol LT (Cognis) erhältlichen Typen; Polyglycerinester und alkoxylierte Polyglycerinester, wie beispielsweise Poly(3)glycerindiisostearat (Handelsprodukt: Lameform TGI (Henkel)) und Poly(2)glycerinpolyhydroxy-stearat (Handelsprodukt: Dehymuls PGPH (Henkel)); Polyolfettsäureester, wie beispielsweise das Handelsprodukt Hydagen HSP (Cognis) oder Sovermol-Typen (Cognis); höher alkoxylierte, propoxylierte und insbesondere ethoxylierte, Mono-, Di- und Triglyceride mit Alkoxylierungsgrad von größer als 5, wie beispielsweise Glycerinmonolaurat + 20 Ethylenoxid und Glycerinmonostearat + 20 Ethylenoxid; Aminoxide; Hydroxymischether; Sorbitanfettsäureester und Anlagerungeprodukte von Ethylenoxid an Sorbitanfettsäureester wie beispielsweise die Polysorbate und Sorbitanmonolaurat + 20 Mol Ethylenoxid (EO); Zuckerfettsäureester und Anlagerungsprodukte von Ethylenoxid an Zuckerfettsäureester; Anlagerungsprodukte von Ethylenoxid an Fettsäurealkanolamide und Fettamine; Fettsäure-N-alkylglucamide; Alkylphenole und Alkylphenolalkoxylate mit 6 bis 21, insbesondere 6 bis 15 Kohlenstoffatomen in der Alkylkette und 5 bis 30 Ethylenoxid- und/oder Propylenoxid-Einheiten; Alkylpolyglykoside entsprechend der allgemeinen Formel RO-(Z)ₓ, wobei R für Alkyl, Z für Zucker sowie x für die Anzahl der Zuckereinheiten steht.

Zu den nichtionischen Emulgatoren im Sinne der Erfindung zählen weiterhin die Polymerisationsprodukte aus Ethylenoxid und Propylenoxid an gesättigte oder ungesättigte Fettalkohole; Fettsäureester mehrwertiger Alkohole mit gesättigten oder ungesättigten Fettsäuren; Alkylester von gesättigten oder ungesättigten Fettsäuren oder Alkylphenole und deren Alkoxylate; insbesondere Ethylenglykolether von Fettalkoholen; gemischte Ethylen- und Propylenglykolether mit Fettalkoholen; Fettsäureester an Sorbitan sowie Polyethylenglykol; Ester von nicht hydroxylierten C₆-C₃₀-Alkylmonocarbonäuren mit Polyethylenglykol; und Anlagerungsprodukte von Alkylphenolen an Ethylen- und/oder Propylenoxid.

Weiterhin kann es zur Trennung der hydrophilen und hydrophoben Phase im erfindungsgemäßen Mittel vorteilhaft sein, zusätzlich dem Mittel Elektrolyte zuzugeben. Unter Elektrolyten werden üblicherweise geladene, ionische anorganische und organische Verbindungen verstanden, welche über keinen oder nur einen nur sehr gering ausgeprägten hydrophoben Anteil enthalten. Bevorzugte Elektrolyte sind gut wasserlösliche Salze, insbesondere Alkalimetall- und Erdalkalimetallsalze von Mineralsäuren und organischen Säuren. Beispiele hierfür sind Natriumchlorid, Natriumsulfat, Natriumhydrogensulfat, Natriumcarbonat, Natriumhydrogencarbonat, Natriumcitrat, Magnesiumchlorid, Magnesiumsulfat, Magnesiumcarbonat und Magnesiumhydrogencarbonat.

Das erfindungsgemäße Mittel zeichnet sich dadurch aus, dass sich öllösliche Inhaltsstoffe überwiegend in der Siliconöl-haltigen Phase (II) anreichern und daher nicht in unmittelbaren Kontakt mit der Oxidationsmittel-haltigen Phase (I) kommen. Dies ist besonders vorteilhaft, um oxidativ wenig stabile Pflegestoffe im Mittel zu stabilisieren. Solche Pflegestoffe sind daher öllösliche Pflegestoffe, öllösliche Vitamine und Triglyceride, insbesondere solche, die eine oder mehrere ungesättigte Kohlenstoff-Kohlenstoff-Bindungen enthalten.

Erfindungsgemäße Mittel sind daher dadurch gekennzeichnet, dass sie mindestens eine überwiegend im Siliconöl lösliche Pflegekomponente, ausgewählt aus öllöslichen Pflegestoffen, öllöslichen Vitaminen und Triglyceriden, enthalten.

Öllösliche Pflegestoffe sind beispielsweise kosmetisch wirksame Terpene und Terpenoide, wie beispielsweise Bisabolol, und Ubichinone, wie beispielsweise Coenzym Q-10.

Öllösliche Vitamine sind insbesondere die Verbindungen, die unter den Sammelbezeichnungen Vitamin A, Vitamin D, Vitamin E und Vitamin K bekannt sind. Ein erfindungsgemäß bevorzugtes Mittel enthält daher mindestens ein öllösliches Vitamin, ausgewählt aus Vitamin A, Vitamin D, Vitamin E und/oder Vitamin K sowie Vitamin P. Vitamin A umfasst dabei Retinoide, insbesondere *all-trans-*Retinol. Vitamin D, auch als Calciferole bezeichnet, umfasst 7,8-Didehydrosterol-Derivate, insbesondere die Verbindungen mit der Bezeichnung Cholecalciferol (Vitamin D₃, Calciol), Ergocalciferol (Vitamin D₂, Ercalciol), 7,8-Didehydrocholesterol (Provitamin D₃, Procalciol, Procholecalciferol) und Ergosterol (Provitamin D₂). Weitere, einsetzbare Vitamin D-Analoga sind Calcidiol (25-Hydroxycholecalciferol), Calcitriol, Hydroxycalcidiol und Vitamin D₁ (Ergocalciferol und Lumisterol). Vitamin E ist die Sammelbezeichnung für Tocopherole und umfasst insbesondere die chemischen Verbindungen α-Tocopherol, β-Tocopherol, γ-Tocopherol, δ-Tocopherol und α-Tocotrienol, β-Tocotrienol, γ-Tocotrienol und δ-Tocotrienol. Vitamin K ist Sammelbezeichnung für verschiedene Verbindungen mit Vitamin-K-Aktivität, die sich von 2-Methyl-1,4-naphthochinon (Vitamin K₃) ableiten. Bevorzugte Vertreter sind Vitamin K₁₍₂₀₎ (2-Methyl-3-phytyl-1,4-naphthochinon), Phyllochinon (Kurzzeichen: K),], Vitamin K₂₍₃₅₎ (3-all-trans-Farnesylgeranylgeranyl-2-methyl-1,4-naphthochinon), Vitamin K₃ (2-Methyl-1,4-naphthochinon, Menadion, Menaphthon) sowie die abgeleiteten Analoga Vitamin K₄ (2-Methyl-1,4-naphthalindiol), Vitamin K₅ (4-Amino-2-methyl-1-naphthol), Vitamin K₆ (2-Methyl-1,4-naphthalindiamin) und Vitamin K₇ (4-Amino-3-methyl-1-naphthol). Bei Vitamin P handelt es sich um eine Sammelbezeichnung für Rutine, insbesondere Bioflavonoide wie Troxerutin (Vitamin P₄) und Hesperidin.

Triglyceride sind die Sammelbezeichnung für Ester des Glycerins, welche die Hauptbestandteile natürlicher Öle darstellen. Erfindungsgemäß besonders bevorzugte Triglyceride sind solche, die zumindest einen Ester einer ungesättigten Fettsäure enthalten. Bevorzugte, ungesättigte Fettsäuren sind Ölsäure, Linolsäure und Linolensäure.

Die erfindungsgemäßen Mittel dienen bevorzugt zur Farbveränderung keratinischer Fasern. Dazu wird das erfindungsgemäße, zweiphasige Mittel (M1) mit einem weiteren Mittel (M2), enthaltend mindestens eine farbverändernde Komponente, vermischt und die resultierende, anwendungsbereite Zubereitung auf die keratinische Fasern gegeben.

Als farbverändernde Komponente in Mittel (M2) dienen als Aufhellmittel zusätzliche Bleichkraftverstärker, die die Wirkung des Oxidationsmittels aus Phase (I) des zweiphasigen Mittels verstärken, sowie farbgebende Komponenten.

In einer Ausführungsform enthält das erfindungsgemäße Mittel (M2) daher einen zusätzlichen Bleichkraftverstärker. Als zusätzliche Bleichkraftverstärker können im Rahmen dieser Erfindung Peroxoverbindungen, weiterhin Verbindungen, die unter Perhydrolysebedingungen aliphatische Peroxocarbonsäuren und/oder substituierte Perbenzoesäure ergeben, Kohlensäurederivate, Alkylcarbonate, -carbamate, Silylcarbonate und -carbamate eingesetzt werden.

Vorzugsweise ist der Bleichkraftverstärker ausgewählt aus Ammoniumperoxodisulfat, Alkalimetallperoxodisulfaten, Ammoniumperoxomonosulfat, Alkalimetallhydrogenperoxomonosulfaten, Alkalimetallperoxodiphosphaten und Erdalkalimetallperoxiden. Besonders bevorzugte Bleichkraftverstärker sind Ammoniumperoxodisulfat, Kaliumperoxodisulfat, Natriumperoxodisulfat, Kaliumhydrogenperoxomonosulfat, Kaliumperoxodiphosphat, Magnesiumperoxid und Bariumperoxid. Erfindungsgemäß besonders bevorzugt sind Mittel, die als Bleichkraftverstärker mindestens ein anorganisches Salz, ausgewählt aus Peroxomonosulfaten und/oder Peroxodisulfaten, enthalten. Weiter hin hat es sich bei den Arbeiten zur vorliegenden Erfindung als besonders bevorzugt erwiesen, wenn die erfindungsgemäßen Mittel mindestens zwei verschiedene Peroxodisulfate enthalten. Bevorzugte Peroxodisulfatsalze sind dabei Kombinationen aus Ammoniumperoxodisulfat und Kaliumperoxodisulfat und/oder Natriumperoxodisulfat. Die Peroxoverbindungen sind in einer Menge von 0,1 bis 25 Gew.-%, insbesondere in einer Menge von 0,5 bis 15 Gew.-%, bezogen auf das Gesamtgewicht des anwendungsbereiten Mittels, enthalten.

Der Einsatz von Persulfatsalzen bzw. Peroxodisulfatsalzen erfolgt in der Regel wasserfrei und in Form eines gegebenenfalls entstaubten Pulvers, Paste oder eines in Form gepressten Formkörpers. Die wasserfreien Mittel (M2) können anstelle und/oder zusätzlich zu den festen Peroxoverbindungen einen weiteren Bleichkraftverstärker enthalten.

Als Bleichverstärker können Verbindungen, die unter Perhydrolysebedingungen aliphatische Peroxocarbonsäuren mit vorzugsweise 1 bis 10 C-Atomen, insbesondere 2 bis 4 C-Atomen, und/oder gegebenenfalls substituierte Perbenzoesäure ergeben, eingesetzt werden. Geeignet sind Substanzen, die O- und/oder N-Acylgruppen der genannten C-Atomzahl und/oder gegebenenfalls substituierte Benzoylgruppen tragen. Bevorzugt sind mehrfach acylierte Alkylendiamine, insbesondere Tetraacetylethylendiamin (TAED), acylierte Triazinderivate, insbesondere 1,5-Diacetyl-2,4-dioxohexahydro-1,3,5-triazin (DADHT), acylierte Glykolurile, insbesondere Tetraacetylglykoluril (TAGU), N-Acylimide, insbesondere N-Nonanoylsuccinimid (NOSI), acylierte Phenolsulfonate, insbesondere n-Nonanoyl- oder Isononanoyloxybenzolsulfonat (n- bzw. i-NOBS), Carbonsäureanhydride, insbesondere Phthalsäureanhydrid, acylierte mehrwertige Alkohole, insbesondere Triacetin, Ethylenglykoldiacetat und 2,5-Diacetoxy-2,5-dihydrofuran.

Als Bleichverstärker vom Typ der Kohlensäurederivate können erfindungsgemäß bevorzugt Carbonatsalze bzw. Hydrogencarbonatsalze eingesetzt werden. Diese sind bevorzugt ausgewählt aus der Gruppe der Ammonium-, Alkalimetall- (insbesondere Natrium- und Kalium-) sowie Erdalkalimetall- (insbesondere Magnesium- und Calcium-), -carbonatsalze bzw. -hydrogencarbonatsalze. Besonders bevorzugte Carbonat- bzw. Hydrogencarbonatsalze sind Ammoniumhydrogencarbonat, Ammoniumcarbonat, Natriumhydrogencarbonat, Natriumcarbonat, Kaliumhydrogencarbonat, Kaliumcarbonat, Magnesiumcarbonat und Calciumcarbonat. Diese besonders bevorzugten Salze können allein oder in deren Mischungen von mindestens zwei Vertretern als Bleichkraftverstärker verwendet werden.

Obwohl prinzipiell keine Einschränkungen hinsichtlich der Formulierung der weiteren Mittel (M2) bestehen, hat es sich erfindungsgemäß als bevorzugt erwiesen, wenn Mittel (M2) wasserfrei formuliert ist. Wasserfrei im Sinne der vorliegenden Erfindung bedeutet einen Wassergehalt bezogen auf die Mittel (M2) von weniger als 5 Gew.-%, insbesondere von weniger als 2 Gew.-%. Blondierzubereitungen, die weniger als 0,1 Gew.-% Wasser, enthalten können erfindungsgemäß ganz besonders bevorzugt sein. Mittel (M2) ist vorzugsweise als Pulver oder als wasserfreie Paste formuliert.

Bleichverstärker vom Typ der Alkylcarbonate und -carbamate sowie Silylcarbonate und Silylcarbamate können in den wasserfreien Zusammensetzungen als Bleichverstärker eingesetzt werden und sind durch Verbindungen der Formel (BV) gekennzeichnet 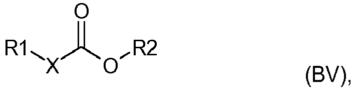
worin R1 für einen gesättigten oder ungesättigten, geradkettigen, verzweigten, oder cyclischen, substituierten oder unsubstituierten Kohlenwasserstoffrest, oder eine substituierte oder unsubstituierte Arylgruppe bzw. einen substituierten oder unsubstituierten Heterocyclus steht,
X für eine Gruppe O oder NR3 steht, worin R3 für ein Wasserstoffatom, einen gesättigten oder ungesättigten, geradkettigen, verzweigten, oder cyclischen, substituierten oder unsubstituierten Kohlenwasserstoffrest oder für eine substituierte oder unsubstituierte Silylgruppe oder für eine substituierte oder unsubstituierte Arylgruppe bzw. einen substituierten oder unsubstituierten Heterocyclus steht, und
R2 für ein Wasserstoffatom, ein Alkalimetallatom, insbesondere Natrium, oder eine Gruppe SiR₃ in der die Reste R unabhängig voneinander für ein Wasserstoffatom, einen gesättigten oder ungesättigten, geradkettigen, verzweigten, oder cyclischen, substituierten oder unsubstituierten Kohlenwasserstoffrest oder für eine Trialkylsilylgruppe, vorzugsweise eine Trimethylsilylgruppe oder für eine substituierte oder unsubstituierte Arylgruppe bzw. einen substituierten oder unsubstituierten Heterocyclus oder für ein Halogen, eine substituierte oder unsubstituierte Hydroxy- oder Aminogruppe stehen.

In Formel (BV) steht R1 vorzugsweise für einen substituierten oder unsubstituierten, geradkettigen oder verzweigten Alkyl-, Alkenyl- oder Alkinylrest, wobei als Substituenten bevorzugt Hydroxy-, Amino-, Nitro-, Sulfonsäuregruppen oder Halogene in Frage kommen. Weitere bevorzugte Reste R1 sind Phenyl- und Benzylreste sowie weiter substituierte Vertreter. Erfindungsgemäß besonders bevorzugt verwendete Zusammensetzungen sind dadurch gekennzeichnet, dass der Rest R1 in Formel (BV) ausgewählt ist aus Methyl, Ethyl, n-Propyl, i-Propyl, n-Butyl, i-Butyl, t-Butyl sowie Hydroxymethyl und Hydroxyethyl. Bevorzugte Reste R2 und R3 in der Formel (BV) sind Wasserstoff, substituierte oder unsubstituierte, geradkettige oder verzweigte Alkylreste sowie Trialkylsilylreste. Unter diesen sind Wasserstoff, Methyl-, Ethyl-, t-Butyl- und Trimethylsilylreste bevorzugt.

Als weitere zusätzliche Bleichverstärker können in den erfindungsgemäßen Zusammensetzungen bevorzugt mindestens eine Verbindung ausgewählt aus Essigsäure, Milchsäure, Weinsäure, Zitronensäure, Salicylsäure und ortho-Phthalsäure, enthalten sein.

In einer weiteren, bevorzugten Ausführungsform kann das Mittel (M2) als Bleichkraftverstärker mindestens ein kationisches Pyridinium-Derivat enthalten. Bevorzugte Verbindungen sind 4-AcylPyridinium-Derivate und 2-Acylpyridinium-Derivate. Insbesondere bevorzugt sind dabei 2-Acetyl-1-methylpyridinium-p-toluolsulfonat und 4-Acetyl-1-methylpyridinium-p-toluolsulfonat. Weiterhin bevorzugte kationische Pyridinium-Derivate sind dabei kationische 3,4-Dihydroisochinolinium-Derivat. Insbesondere bevorzugt ist N-Methyl-3,4-dihydroisochinolinium-p-toluolsulfonat.

Die neben oder anstelle von Peroxoverbindungen eingesetzten Bleichkraftverstärker sind in den erfindungsgemäßen, kosmetischen Mitteln bevorzugt in Mengen von 0,05 bis 10 Gew.-%, insbesondere in Mengen von 0,2 bis 5 Gew.-%, jeweils bezogen auf das Gesamtgewicht des anwendungsbereiten Mittels, enthalten.

Zur weiteren Steigerung der Aufhellleistung können der erfindungsgemäßen Zusammensetzung zusätzlich als Bleichverstärker mindestens eine gegebenenfalls hydratisierte SiO₂-Verbindun zugesetzt. Obwohl bereits geringe Mengen der gegebenenfalls hydratisierten SiO₂-Verbindungen die Aufhellleistung erhöhen, kann es erfindungsgemäß bevorzugt sein, die gegebenenfalls hydratisierten SiO₂-Verbindungen in Mengen von 0,05 Gew.-% bis 15 Gew.-%, besonders bevorzugt in Mengen von 0,15 Gew.-% bis 10 Gew.-% und ganz besonders bevorzugt in Mengen von 0,2 Gew.-% bis 5 Gew.-%, jeweils bezogen auf die erfindungsgemäße wasserfreie Zusammensetzung, einzusetzen. Die Mengenangaben geben dabei jeweils den Gehalt der SiO₂-Verbindungen (ohne deren Wasseranteil) in den Mitteln wieder.

Bevorzugte gegebenenfalls hydratisierten SiO₂-Verbindungen sind Kieselsäuren, deren Oligomeren und Polymeren sowie deren Salze. Bevorzugte Salze sind die Alkalisalze, insbesondere die Kalium und Natriumsalze. Die Natriumsalze sind ganz besonders bevorzugt. Die gegebenenfalls hydratisierten SiO₂-Verbindungen können in verschiedenen Formen vorliegen. Erfindungsgemäß bevorzugt werden die SiO₂-Verbindungen in Form von Kieselgelen (Silicagel) oder besonders bevorzugt als Wasserglas eingesetzt. Diese SiO₂-Verbindungen können teilweise in wässriger Lösung vorliegen. Erfindungsgemäß ganz besonders bevorzugt sind Wassergläser, die aus einem Silikat der Formel (SiO₂)ₙ(Na₂O)ₘ(K₂O)ₚ gebildet werden, wobei n steht für eine positive rationale Zahl und m und p stehen unabhängig voneinander für eine positive rationale Zahl oder für 0, mit den Maßgaben, dass mindestens einer der Parameter m oder p von 0 verschieden ist und das Verhältnis zwischen n und der Summe aus m und p zwischen 1:4 und 4:1 liegt. Insbesondere Metasilicate, die sich gemäß vorstehender Formel durch das Verhältnis zwischen n und der Summe aus m und p von ≤ 1 auszeichnen und sich als kettenförmige polymere Strukturen des Anions [SiO₃]²⁻ auffassen lassen, können bevorzugt eingesetzt werden. Natriummetasilicat der Formel [NaSiO₃]ₓ, ist dabei besonders bevorzugt.

Neben den durch die Summenformel beschriebenen Komponenten können die Wassergläser in geringen Mengen noch weitere Zusatzstoffe, wie beispielsweise Phosphate oder Magnesiumsalze, enthalten.

In einer weiteren Ausführungsform der vorliegenden Erfindung enthält das Mittel (M2) als farbverändernde Komponente farbgebende Komponenten. Die erfindungsgemäßen Mittel können daher zusätzlich mindestens eine farbgebende Komponente enthalten, die bevorzugt ausgewählt wird
(1) aus mindestens einem Oxidationsfarbstoffvorprodukt und/oder
(2) aus mindestens einem direktziehenden Farbstoff.

Erfindungsgemäß bevorzugte Mittel zum Farbveränderung keratinischer Fasern sind demnach dadurch gekennzeichnet, dass sie mindestens ein Oxidationsfarbstoffvorprodukt enthalten.

Die erfindungsgemäßen Aufhellmittel enthalten als Oxidationsfarbstoffvorprodukt mindestens ein Oxidationsfarbstoffvorprodukt vom Entwicklertyp (Entwicklerkomponente), bevorzugt in Kombination mit mindestens einer Oxidationsfarbstoffvorprodukt vom Kupplertyp (Kupplerkomponente).

Bevorzugte Oxidationsfarbstoffvorprodukte vom Entwicklertyp sind p-Phenylendiaminderivate. Besonders bevorzugte p-Phenylendiamine werden ausgewählt aus einer oder mehrerer Verbindungen der Gruppe, die gebildet wird, aus p-Phenylendiamin, p-Toluylendiamin, 2-Chlor-p-phenylendiamin, 2,3-Dimethyl-p-phenylendiamin, 2,6-Dimethyl-p-phenylendiamin, 2,6-Diethyl-p-phenylendiamin, 2,5-Dimethyl-p-phenylendiamin, N,N-Dimethyl-p-phenylendiamin, N,N-Diethyl-p-phenylendiamin, N,N-Dipropyl-p-phenylendiamin, 4-Amino-3-methyl-(N,N-diethyl)anilin, N,N-Bis-(2-hydroxyethyl)-p-phenylendiamin, 4-N,N-Bis-(2-hydroxyethyl)amino-2-methylanilin, 4-N,N-Bis-(2-hydroxyethyl)-amino-2-chloranilin, 2-(2-Hydroxyethyl)-p-phenylendiamin, 2-(1,2-Dihydroxy-ethyl)-p-phenylendiamin, 2-Fluor-p-phenylendiamin, 2-lsopropyl-p-phenylendiamin, N-(2-Hydroxypropyl)-p-phenylendiamin, 2-Hydroxymethyl-p-phenylendiamin, N,N-Dimethyl-3-methyl-p-phenylendiamin, N-Ethyl-N-2-hydroxyethyl-p-phenylendiamin, N-(2,3-Dihydroxypropyl)-p-phenylendiamin, N-(4'-Aminophenyl)-p-phenylendiamin, N-Phenyl-p-phenylendiamin, 2-(2-Hydroxyethyloxy)-p-phenylendiamin, 2-Methoxymethyl-p-phenylendiamin, 2-(2-Acetylamino-ethyloxy)-p-phenylendiamin, N-(2-Methoxyethyl)-p-phenylendiamin, N-(4-Amino-3-methylphenyl)-N-[3-(1H-imidazol-1-yl)propyl]amin, 5,8-Diaminobenzo-1,4-dioxan sowie ihren physiologisch verträglichen Salzen. Erfindungsgemäß ganz besonders bevorzugte p-Phenylendiaminderivate sind ausgewählt aus mindestens einer Verbindung der Gruppe p-Phenylendiamin, p-Toluylendiamin, 2-(2-Hydroxyethyl)-p-phenylendiamin, 2-(1,2-Dihydroxyethyl)-p-phenylendiamin, N,N-Bis-(2-hydroxyethyl)-p-phenylendiamin, N-(4-Amino-3-methylphenyl)-N-[3-(1H-imidazol-1-yl)propyl]amin, 2-Methoxymethyl-p-phenylendiamin sowie den physiologisch verträglichen Salzen dieser Verbindungen.

Es kann erfindungsgemäß weiterhin bevorzugt sein, als Entwicklerkomponente Verbindungen einzusetzen, die mindestens zwei aromatische Kerne enthalten, die mit Amino- und/oder Hydroxylgruppen substituiert sind. Bevorzugte zweikernige Entwicklerkomponenten werden insbesondere aus mindestens einer der folgenden Verbindungen ausgewählt: N,N'-Bis-(2-hydroxyethyl)-N,N'-bis-(4'-aminophenyl)-1,3-diaminopropan-2-ol, N,N'-Bis-(2-hydroxyethyl)-N,N'-bis-(4'-aminophenyl)-ethylendiamin, N,N'-Bis-(4'-aminophenyl)tetramethylendiamin, N,N'-Bis-(2-hydroxyethyl)-N,N'-bis-(4'-aminophenyl)-tetramethylendiamin, N,N'-Bis-(4-(methylamino)phenyl)tetramethylendiamin, N,N'-Diethyl-N,N'-bis-(4'-amino-3'-methylphenyl)ethylendiamin, Bis-(2-hydroxy-5-aminophenyl)-methan, N,N'-Bis-(4'-aminophenyl)-1,4-diazacycloheptan, N,N'-Bis-(2-hydroxy-5-aminobenzyl)-piperazin, N-(4'-Aminophenyl)-p-phenylendiamin und 1,10-Bis-(2',5'-diaminophenyl)-1,4,7,10-tetraoxadecan sowie ihre physiologisch verträglichen Salze. Ganz besonders bevorzugte zweikernige Entwicklerkomponenten werden ausgewählt unter N,N'-Bis-(2-hydroxyethyl)-N,N'-bis-(4-aminophenyl)-1,3-diamino-propan-2-ol, Bis-(2-hydroxy-5-aminophenyl)methan, 1,3-Bis-(2,5-di-aminophenoxy)-propan-2-ol, N,N'-Bis-(4-aminophenyl)-1,4-diazacycloheptan, 1,10-Bis-(2,5-di-aminophenyl)-1,4,7,10-tetraoxadecan oder eines der physiologisch verträglichen Salze dieser Verbindungen.

Weiterhin kann es erfindungsgemäß bevorzugt sein, als Entwicklerkomponente ein p-Aminophenolderivat oder eines seiner physiologisch verträglichen Salze einzusetzen. Bevorzugte p-Aminophenole sind insbesondere p-Aminophenol, N-Methyl-p-aminophenol, 4-Amino-3-methyl-phenol, 4-Amino-3-fluorphenol, 2-Hydroxymethylamino-4-aminophenol, 4-Amino-3-hydroxy-methylphenol, 4-Amino-2-(2-hydroxyethoxy)-phenol, 4-Amino-2-methylphenol, 4-Amino-2-hydroxymethylphenol, 4-Amino-2-methoxymethyl-phenol, 4-Amino-2-aminomethylphenol, 4-Amino-2-(2-hydroxyethyl-aminomethyl)-phenol, 4-Amino-2-(1,2-dihydroxyethyl)-phenol, 4-Amino-2-fluorphenol, 4-Amino-2-chlorphenol, 4-Amino-2,6-dichlorphenol, 4-Amino-2-(diethylamino-methyl)phenol sowie ihre physiologisch verträglichen Salze. Ganz besonders bevorzugte Verbindungen sind p-Aminophenol, 4-Amino-3-methylphenol, 4-Amino-2-aminomethylphenol, 4-Amino-2-(1,2-dihydroxyethyl)phenol und 4-Amino-2-(diethylaminomethyl)phenol.

Ferner kann die Entwicklerkomponente ausgewählt sein aus o-Aminophenol und seinen De-rivaten, wie beispielsweise 2-Amino-4-methylphenol, 2-Amino-5-methylphenol oder 2-Amino-4-chlorphenol.

Weiterhin kann die Entwicklerkomponente ausgewählt sein aus heterocyclischen Entwicklerkomponenten, wie beispielsweise aus Pyrimidinderivaten, Pyrazolderivaten, Pyrazolopyrimidin-und Pyrazolopyrazol-Derivaten bzw. ihren physiologisch verträglichen Salzen.

Besonders bevorzugte Pyrimidin-Derivate sind insbesondere die Verbindungen 2,4,5,6-Tetraaminopyrimidin, 4-Hydroxy-2,5,6-triaminopyrimidin, 2-Hydroxy-4,5,6-triaminopyrimidin, 2-Dimethylamino-4,5,6-triaminopyrimidin, 2,4-Dihydroxy-5,6-diaminopyrimidin und 2,5,6-Triaminopyrimidin.

Besonders bevorzugte Pyrazol-Derivate sind insbesondere die Verbindungen, die ausgewählt werden unter 4,5-Diamino-1-methylpyrazol, 4,5-Diamino-1-(2-hydroxyethyl)pyrazol, 3,4-Diaminopyrazol, 4,5-Diamino-1-(4'-chlorbenzyl)pyrazol, 4,5-Diamino-1,3-dimethylpyrazol, 4,5-Diamino-3-methyl-1-phenylpyrazol, 4,5-Diamino-1-methyl-3-phenylpyrazol, 4-Amino-1,3-dimethyl-5-hydrazinopyrazol, 1-Benzyl-4,5-diamino-3-methylpyrazol, 4,5-Diamino-3-t-butyl-1-methylpyrazol, 4,5-Diamino-1-t-butyl-3-methylpyrazol, 4,5-Diamino-1-(2-hydroxyethyl)-3-methylpyrazol, 4,5-Diamino-1-ethyl-3-methylpyrazol, 4,5-Diamino-1-ethyl-3-(4-methoxyphenyl)pyrazol, 4,5-Diamino-1-ethyl-3-hydroxymethylpyrazol, 4,5-Diamino-3-hydroxymethyl-1-methylpyrazol, 4,5-Diamino-3-hydroxymethyl-1-isopropylpyrazol, 4,5-Diamino-3-methyl-1-isopropylpyrazol, 4-Amino-5-(2-aminoethyl)-amino-1,3-dimethylpyrazol, sowie deren physiologisch verträglichen Salze, insbesondere jedoch 4,5-Diamino-1-(2-hydroxyethyl)pyrazol.

Bevorzugte Pyrazolopyrimidine sind insbesondere die Verbindungen, die ausgewählt werden unter
Pyrazolo[1,5-a]pyrimidin-3,7-diamin, 2,5-Dimethyl-pyrazolo[1,5-a]pyrimidin-3,7-diamin, Pyrazolo-[1,5-a]pyrimidin-3,5-diamin, 2,7-Dimethyl-pyrazolo[1,5-a]pyrimidin-3,5-diamin, 3-Aminopyrazolo-[1,5-a]pyrimidin-7-ol, 3-Aminopyrazolo[1,5-a]pyrimidin-5-ol, 2-(3-Aminopyrazolo[1,5-a]pyrimidin-7-ylamino)ethanol, 2-(7-Aminopyrazolo[1,5-a]pyrimidin-3-ylamino)ethanol, 2-[(3-Aminopyrazolo[1,5-a]pyrimidin-7-yl)-(2-hydroxy-ethyl)-amino]ethanol, 2-[(7-Aminopyrazolo[1,5-a]pyrimidin-3-yl)-(2-hydroxyethyl)amino]ethanol, 5,6-Dimethylpyrazolo[1,5-a]pyrimidin-3,7-diamin, 2,6-Dimethylpyrazolo[1,5-a]pyrimidin-3,7-diamin, 3-Amino-7-dimethylamino-2,5-dimethylpyrazolo[1,5-a]-pyrimidin sowie ihre physiologisch verträglichen Salze und ihre tautomeren Formen, wenn ein tautomeres Gleichgewicht vorhanden ist. Eine bevorzugtes Pyrazolopyrazol-Derivat ist insbesondere 2,3-Diamino-6,7-dihydro-1H,5H-pyrazolo[1,2-a]pyrazol-1-on.

Besonders bevorzugte Entwicklerkomponenten werden ausgewählt aus mindestens einer Verbindung aus der Gruppe, die gebildet wird aus p-Phenylendiamin, p-Toluylendiamin, 2-(2-Hydroxyethyl)-p-phenylendiamin, 2-(1,2-Dihydroxyethyl)-p-phenylendiamin, N,N-Bis-(2-hydroxyethyl)-p-phenylendiamin, 2-Methoxymethyl-p-phenylendiamin, N-(4-Amino-3-methylphenyl)-N-[3-(1H-imidazol-1-yl)propyl]amin, N,N'-Bis-(2-hydroxyethyl)-N,N'-bis-(4-aminophenyl)-1,3-diaminopropan-2-ol, Bis-(2-hydroxy-5-aminophenyl)methan, 1,3-Bis-(2,5-diaminophenoxy)propan-2-ol, N,N'-Bis-(4-aminophenyl)-1,4-diazacycloheptan, 1,10-Bis-(2,5-diaminophenyl)-1,4,7,10-tetraoxadecan, p-Aminophenol, 4-Amino-3-methylphenol, 4-Amino-2-aminomethylphenol, 4-Amino-2-(1,2-dihydroxyethyl)phenol und 4-Amino-2-(diethylaminomethyl)phenol, 4,5-Diamino-1-(2-hydroxyethyl)pyrazol, 2,4,5,6-Tetraaminopyrimidin, 4-Hydroxy-2,5,6-triaminopyrimidin, 2-Hydroxy-4,5,6-triaminopyrimidin, 2,3-Diamino-6,7-dihydro-1H,5H-pyrazolo[1,2-a]pyrazol-1-on sowie den physiologisch verträglichen Salzen dieser Verbindungen.

Ganz besonders bevorzugte Entwicklerkomponenten sind dabei p-Toluylendiamin, 2-(2-Hydroxyethyl)-p-phenylendiamin, 2-Methoxymethyl-p-phenylendiamin, N-(4-Amino-3-methylphenyl)-N-[3-(1H-imidazol-1-yl)propyl]amin, und/oder 4,5-Diamino-1-(2-hydroxyethyl)-pyrazol sowie deren physiologisch verträglichen Salze.

Die Entwicklerkomponenten werden bevorzugt in einer Menge von 0,0001 bis 0,5 Gew.-%, vorzugsweise 0,001 bis 0,2 Gew.-%, jeweils bezogen auf das anwendungsbereite Mittel, verwendet.

Kupplerkomponenten bilden im Rahmen der oxidativen Färbung allein keine signifikante Färbung aus, sondern benötigen stets die Gegenwart von Entwicklerkomponenten. Daher ist es erfindungsgemäß bevorzugt, dass bei Verwendung mindestens einer Kupplerkomponente zusätzlich mindestens eine Entwicklerkomponente zum Einsatz kommt. Kupplerkomponenten im Sinne der Erfindung erlauben mindestens eine Substitution eines chemischen Restes des Kupplers durch die oxidierte Form der Entwicklerkomponente. Dabei bildet sich eine kovalente Bindung zwischen Kuppler- und Entwicklerkomponente aus. Kuppler sind bevorzugt cyclische Verbindungen, die am Cyclus mindestens zwei Gruppen tragen, ausgewählt aus (i) gegebenenfalls substituierten Aminogruppen und/oder (ii) Hydroxylgruppen. Wenn die cyclische Verbindung ein Sechsring (bevorzugt aromatisch) ist, so befinden sich die besagten Gruppen bevorzugt in ortho-Position oder meta-Position zueinander.

Erfindungsgemäße Kupplerkomponenten werden bevorzugt als mindestens eine Verbindung aus einer der folgenden Klassen ausgewählt: m-Aminophenol, o-Aminophenol, m-Diaminobenzol, o-Diaminobenzol und/oder deren Derivate; Naphthalinderivate mit mindestens einer Hydroxygruppe; Di- beziehungsweise Trihydroxybenzol; Pyridinderivate; Pyrimidinderivate; bestimmte Indol-Derivate und Indolin-Derivate; Pyrazolonderivate (beispielsweise 1-Phenyl-3-methylpyrazol-5-on); Morpholinderivate (beispielsweise 6-Hydroxybenzomorpholin oder 6-Aminobenzomorpholin); Chinoxalinderivate (beispielsweise 6-Methyl-1,2,3,4-tetrahydrochinoxalin), sowie Gemische aus zwei oder mehreren Verbindungen aus einer oder mehreren dieser Klassen.

Besonders bevorzugte m-Aminophenol-Kupplerkomponenten werden ausgewählt aus mindestens einer Verbindung aus der Gruppe, die gebildet wird aus 3-Aminophenol, 5-Amino-2-methylphenol, N-Cyclopentyl-3-aminophenol, 3-Amino-2-chlor-6-methylphenol, 2-Hydroxy-4-aminophenoxy-ethanol, 2,6-Dimethyl-3-aminophenol, 3-Trifluoroacetylamino-2-chlor-6-methylphenol, 5-Amino-4-chlor-2-methylphenol, 5-Amino-4-methoxy-2-methylphenol, 5-(2'-Hydroxyethyl)amino-2-methylphenol, 3-Diethylaminophenol, N-Cyclopentyl-3-aminophenol, 1,3-Dihydroxy-5-(methylamino)-benzol, 3-Ethylamino-4-methylphenol, 2,4-Dichlor-3-aminophenol und den physiologisch verträglichen Salzen aller vorstehend genannten Verbindungen.

Besonders bevorzugte m-Diaminobenzol-Kupplerkomponenten werden ausgewählt aus mindestens einer Verbindung aus der Gruppe, die gebildet wird aus m-Phenylendiamin, 2-(2,4-Diaminophenoxy)ethanol, 1,3-Bis(2,4-diaminophenoxy)propan, 1-Methoxy-2-amino-4-(2'-hydroxyethylamino)benzol, 1,3-Bis(2,4-diaminophenyl)propan, 2,6-Bis(2'-hydroxyethylamino)-1-methylbenzol, 2-({3-[(2-Hydroxyethyl)amino]-4-methoxy-5-methylphenyl}amino)ethanol, 2-({3-[(2-Hydroxyethyl)-amino]-2-methoxy-5-methylphenyl}amino)ethanol, 2-({3-[(2-Hydroxyethyl)amino]-4,5-dimethylphenyl}amino)ethanol, 2-[3-Morpholin-4-ylphenyl)amino]ethanol, 3-Amino-4-(2-methoxyethoxy)-5-methylphenylamin, 1-Amino-3-bis-(2'-hydroxyethyl)aminobenzol und den physiologisch verträglichen Salzen aller vorstehend genannten Verbindungen.

Besonders bevorzugte o-Diaminobenzol-Kupplerkomponenten werden ausgewählt aus mindestens einer Verbindung aus der Gruppe, die gebildet wird aus 3,4-Diaminobenzoesäure und 2,3-Diamino-1-methylbenzol und den physiologisch verträglichen Salzen aller vorstehend genannten Verbindungen.

Bevorzugte Naphthalinderivate mit mindestens einer Hydroxygruppe werden ausgewählt aus mindestens einer Verbindung der Gruppe, die gebildet wird aus 1-Naphthol, 2-Methyl-1-naphthol, 2-Hydroxymethyl-1-naphthol, 2-Hydroxyethyl-1-naphthol, 1,3-Dihydroxynaphthalin, 1,5-Dihydroxynaphthalin, 1,6-Dihydroxynaphthalin, 1,7-Dihydroxynaphthalin, 1,8-Dihydroxynaphthalin, 2,7-Dihydroxynaphthalin und 2,3-Dihydroxynaphthalin.

Bevorzugte Di- beziehungsweise Trihydroxybenzole und deren Derivate werden ausgewählt aus mindestens einer Verbindung der Gruppe, die gebildet wird aus Resorcin, Resorcinmonomethylether, 2-Methylresorcin, 5-Methylresorcin, 2,5-Dimethylresorcin, 2-Chlorresorcin, 4-Chlorresorcin, Pyrogallol und 1,2,4-Trihydroxybenzol.

Besonders bevorzugte Pyridinderivate werden ausgewählt aus mindestens einer Verbindung der Gruppe, die gebildet wird aus 2,6-Dihydroxypyridin, 2-Amino-3-hydroxypyridin, 2-Amino-5-chlor-3-hydroxypyridin, 3-Amino-2-methylamino-6-methoxypyridin, 2,6-Dihydroxy-3,4-dimethylpyridin, 2,6-Dihydroxy-4-methylpyridin, 2,6-Diaminopyridin, 2,3-Diamino-6-methoxypyridin, 3,5-Diamino-2,6-dimethoxypyridin, 3,4-Diaminopyridin, 2-(2-Methoxyethyl)amino-3-amino-6-methoxypyridin, 2-(4'-Methoxyphenyl)amino-3-aminopyridin, und den physiologisch verträglichen Salzen der vorgenannten Verbindungen.

Bevorzugte Pyrimidinderivate werden ausgewählt aus mindestens einer Verbindung der Gruppe, die gebildet wird aus 4,6-Diaminopyrimidin, 4-Amino-2,6-dihydroxypyrimidin, 2,4-Diamino-6-hydroxypyrimidin, 2,4,6-Trihydroxypyrimidin, 2-Amino-4-methylpyrimidin, 2-Amino-4-hydroxy-6-methylpyrimidin und 4,6-Dihydroxy-2-methylpyrimidin und den physiologisch verträglichen Salzen der vorgenannten Verbindungen.

Besonders bevorzugte Indolderivate werden ausgewählt aus mindestens einer Verbindung der Gruppe, die gebildet wird aus 4-Hydroxyindol, 6-Hydroxyindol und 7-Hydroxyindol und den physiologisch verträglichen Salzen der vorgenannten Verbindungen.

Besonders bevorzugte Indolinderivate werden ausgewählt aus mindestens einer Verbindung der Gruppe, die gebildet wird aus 4-Hydroxyindolin, 6-Hydroxyindolin und 7-Hydroxyindolin und den physiologisch verträglichen Salzen der vorgenannten Verbindungen.

Erfindungsgemäß besonders bevorzugte Kupplerkomponenten werden ausgewählt unter 3-Aminophenol, 5-Amino-2-methylphenol, 3-Amino-2-chlor-6-methylphenol, 2-Hydroxy-4-aminophenoxyethanol, 5-Amino-4-chlor-2-methylphenol, 5-(2-Hydroxyethyl)-amino-2-methylphenol, 2,4-Dichlor-3-aminophenol, 2-Aminophenol, 3-Phenylendiamin, 2-(2,4-Diaminophenoxy)ethanol, 1,3-Bis(2,4-diaminophenoxy)propan, 1-Methoxy-2-amino-4-(2-hydroxyethylamino)benzol, 1,3-Bis(2,4-diaminophenyl)propan, 2,6-Bis(2'-hydroxyethylamino)-1-methylbenzol, 2-({3-[(2-Hydroxyethyl)-amino]-4-methoxy-5-methylphenyl}amino)ethanol, 2-({3-[(2-Hydroxyethyl)amino]-2-methoxy-5-methylphenyl}amino)ethanol, 2-({3-[(2-Hydroxyethyl)amino]-4,5-dimethylphenyl}amino)ethanol, 2-[3-Morpholin-4-ylphenyl)amino]ethanol, 3-Amino-4-(2-methoxyethoxy)-5-methylphenylamin, 1-Amino-3-bis-(2-hydroxyethyl)aminobenzol, Resorcin, 2-Methylresorcin, 4-Chlorresorcin, 1,2,4-Trihydroxybenzol, 2-Amino-3-hydroxypyridin, 3-Amino-2-methylamino-6-methoxypyridin, 2,6-Dihydroxy-3,4-dimethylpyridin, 3,5-Diamino-2,6-dimethoxypyridin, 1-Phenyl-3-methylpyrazol-5-on, 1-Naphthol, 1,5-Dihydroxynaphthalin, 2,7-Dihydroxynaphthalin, 1,7-Dihydroxynaphthalin, 1,8-Dihydroxynaphthalin, 4-Hydroxyindol, 6-Hydroxyindol, 7-Hydroxyindol, 4-Hydroxyindolin, 6-Hydroxyindolin, 7-Hydroxyindolin oder Gemischen dieser Verbindungen oder den physiologisch verträglichen Salzen der vorgenannten Verbindungen.

Ganz besonders bevorzugt ist dabei Resorcin, 2-Methylresorcin, 5-Amino-2-methylphenol, 3-Aminophenol, 2-(2,4-Diaminophenoxy)ethanol, 1,3-Bis(2,4-diaminophenoxy)propan, 1-Methoxy-2-amino-4-(2'-hydroxyethylamino)benzol, 2-Amino-3-hydroxypyridin und 1-Naphthol sowie eines deren physiologisch verträglichen Salze.

Die Kupplerkomponenten werden bevorzugt in einer Menge von 0,0001 bis 0,5 Gew.-%, vorzugsweise 0,001 bis 0,2 Gew.-%, jeweils bezogen auf das anwendungsbereite Mittel, verwendet.

Dabei werden Entwicklerkomponenten und Kupplerkomponenten im Allgemeinen in etwa molaren Mengen zueinander eingesetzt. Wenn sich auch der molare Einsatz als zweckmäßig erwiesen hat, so ist ein gewisser Überschuss einzelner Oxidationsfarbstoffvorprodukte nicht nachteilig, so dass Entwicklerkomponenten und Kupplerkomponenten in einem Mol-Verhältnis von 1 zu 0,5 bis 1 zu 3, insbesondere 1 zu 1 bis 1 zu 2, stehen können.

Weiterhin können die erfindungsgemäßen Mittel mindestens einen direktziehenden Farbstoff enthalten. Dabei handelt sich um Farbstoffe, die direkt auf das Haar aufziehen und keinen oxidativen Prozess zur Ausbildung der Farbe benötigen. Direktziehende Farbstoffe können in anionische, kationische und nichtionische direktziehende Farbstoffe unterteilt werden. Üblicherweise sind es Nitrophenylendiamine, Nitroaminophenole, Azofarbstoffe, Anthrachinone oder Indophenole. Die direktziehenden Farbstoffe werden jeweils bevorzugt in einer Menge von 0,0001 bis 0,2 Gew.-%, bevorzugt von 0,001 bis 0,1 Gew.-%, jeweils bezogen auf die gesamte Anwendungszubereitung, eingesetzt. Die Gesamtmenge an direktziehenden Farbstoffen beträgt vorzugsweise höchstens 0,1 Gew.-%.

Bevorzugte anionische direktziehende Farbstoffe sind die unter den internationalen Bezeichnungen bzw. Handelsnamen Acid Yellow 1, Yellow 10, Acid Yellow 23, Acid Yellow 36, Acid Orange 7, Acid Red 33, Acid Red 52, Pigment Red 57:1, Acid Blue 7, Acid Green 50, Acid Violet 43, Acid Black 1, Acid Black 52, Bromphenolblau und Tetrabromphenolblau bekannten Verbindungen.

Bevorzugte kationische direktziehende Farbstoffe sind kationische Triphenylmethanfarbstoffe, wie beispielsweise Basic Blue 7, Basic Blue 26, Basic Violet 2 und Basic Violet 14, aromatischen Systeme, die mit einer quaternären Stickstoffgruppe substituiert sind, wie beispielsweise Basic Yellow 57, Basic Red 76, Basic Blue 99, Basic Brown 16 und Basic Brown 17, sowie direktziehende Farbstoffe, die einen Heterocyclus enthalten, der mindestens ein quaternäres Stickstoffatom aufweist, insbesondere Basic Yellow 87, Basic Orange 31 und Basic Red 51. Die kationischen direktziehenden Farbstoffe, die unter dem Warenzeichen Arianor^{®} vertrieben werden, sind erfindungsgemäß ebenfalls ganz besonders bevorzugte kationische direktziehende Farbstoffe.

Als nichtionische direktziehende Farbstoffe eignen sich insbesondere nichtionische Nitro- und Chinonfarbstoffe und neutrale Azofarbstoffe. Bevorzugte nichtionische direktziehende Farbstoffe sind die unter den internationalen Bezeichnungen bzw. Handelsnamen HC Yellow 2, HC Yellow 4, HC Yellow 5, HC Yellow 6, HC Yellow 12, HC Orange 1, Disperse Orange 3, HC Red 1, HC Red 3, HC Red 10, HC Red 11, HC Red 13, HC Red BN, HC Blue 2, HC Blue 11, HC Blue 12, Disperse Blue 3, HC Violet 1, Disperse Violet 1, Disperse Violet 4, Disperse Black 9 bekannten Verbindungen, sowie 1,4-Diamino-2-nitrobenzol, 2-Amino-4-nitrophenol, 1,4-Bis-(2-hydroxyethyl)-amino-2-nitrobenzol, 3-Nitro-4-(2-hydroxyethyl)aminophenol, 2-(2-Hydroxyethyl)amino-4,6-dinitrophenol, 4-[(2-Hydroxyethyl)amino]-3-nitro-1-methylbenzol, 1-Amino-4-(2-hydroxyethyl)amino-5-chlor-2-nitrobenzol, 4-Amino-3-nitrophenol, 1-(2'-Ureidoethyl)amino-4-nitrobenzol, 2-[(4-Amino-2-nitrophenyl)amino]-benzoesäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chlor-6-ethylamino-4-nitrophenol.

Erfindungsgemäß bevorzugte Farbstoffkombinationen sind solche, die mindestens die Kombination aus Tetrabromphenolblau und Acid Red 92; Tetrabromphenolblau und Acid Red 98; Tetrabromphenolblau und Acid Red 94; Tetrabromphenolblau und Acid Red 87 oder Tetrabromphenolblau und Acid Red 51.

Erfindungsgemäße, anwendungsbereite Mittel sind vorzugsweise wässrige, fließfähige Zubereitungen. Die erfindungsgemäßen Mittel können weiterhin alle für solche Zubereitungen bekannten Wirk-, Zusatz- und Hilfsstoffe enthalten. Die anwendungsbereiten Mittel als Mischung aus Mittel (M1) und (M2) können dabei oberflächenaktive Substanzen, ausgewählt aus den oben angeführten anionischen, nichtionischen, zwitterionischen und amphoteren Tensiden enthalten.

Erfindungsgemäß bevorzugt sind ebenfalls kationische Tenside vom Typ der quartären Ammoniumverbindungen, der Esterquats und der Amidoamine. Bevorzugte quaternäre Ammoniumverbindungen sind Ammoniumhalogenide, insbesondere Chloride und Bromide, wie Alkyltrimethylammoniumchloride, Dialkyldimethylammoniumchloride und Trialkylmethylammoniumchloride sowie die unter den INCI-Bezeichnungen Quaternium-27 und Quaternium-83 bekannten Imidazolium-Verbindungen. Weitere erfindungsgemäß verwendbare kationische Tenside stellen die quaternisierten Proteinhydrolysate dar. Alkylamidoamine werden üblicherweise durch Amidierung natürlicher oder synthetischer Fettsäuren und Fettsäureschnitte mit Dialkylaminoaminen hergestellt und zeichnen sich neben einer guten konditionierenden Wirkung speziell durch ihre gute biologische Abbaubarkeit aus, wie zum Beispiel Stearamidopropyl-dimethylamin. Ebenfalls bevorzugte Esterquats sind quaternierte Estersalze von Fettsäuren mit Triethanolamin, quaternierte Estersalze von Fettsäuren mit Diethanolalkylaminen und quaternierten Estersalzen von Fettsäuren mit 1,2-Dihydroxypropyldialkylaminen. Solche Produkte werden beispielsweise unter den Warenzeichen Stepantex, Dehyquart und Armocare vertrieben. Die Produkte Armocare VGH-70, ein N,N-Bis(2-Palmitoyloxyethyl)dimethylammoniumchlorid, sowie Dehyquart F-75, Dehyquart C-4046, Dehyquart L80 und Dehyquart AU-35 sind Beispiele für solche Esterquats.

Die kationischen Tenside sind in den erfindungsgemäß verwendeten Mitteln bevorzugt in Mengen von 0,05 bis 10 Gew.-%, bezogen auf das gesamte Mittel, enthalten. Mengen von 0,1 bis 5 Gew.-% sind besonders bevorzugt.

In einer bevorzugten Ausführungsform können nicht-ionische, zwitterionische und/oder amphotere Tenside sowie deren Mischungen bevorzugt sein.

Erfindungsgemäß kann aber die Oxidationsmittelzubereitung auch zusammen mit einem Katalysator auf das Haar aufgebracht werden, der die Oxidation der Farbstoffvorprodukte, z.B. durch Luftsauerstoff, aktiviert. Solche Katalysatoren sind z. B. bestimmte Enzyme, lodide, Chinone oder Metallionen. Hierfür geeignete Enzyme sind z. B. Peroxidasen, die die Wirkung geringer Mengen an Wasserstoffperoxid deutlich verstärken können. Ein Einsatz bestimmter Metallionen oder - komplexe kann ebenfalls bevorzugt sein. Geeignete Metallionen sind beispielsweise Zn²⁺, Cu²⁺, Fe²⁺, Fe³⁺, Mn²⁺, Mn⁴⁺, Li⁺, Mg²⁺, Ca²⁺, Ce⁴⁺, V³⁺, Co²⁺, Ru³⁺ und Al³⁺. Besonders geeignet sind dabei Zn²⁺, Cu²⁺ und Mn²⁺.

Weiterhin hat es sich als vorteilhaft erweisen, wenn die Oxidationsmittelzubereitungen mindestens einen Stabilisator oder Komplexbildner enthalten. Besonders bevorzugte Stabilisatoren sind Phenacetin, Alkalibenzoate (Natriumbenzoat) und Salicylsäure.

Erfindungsgemäß bevorzugt ist auch der Einsatz von sogenannten Komplexbildnern. Komplexbilder sind Stoffe, die Metallionen komplexieren können. Bevorzugte Komplexbildner sind sogenannte Chelatkomplexbildner, also Stoffe, die mit Metallionen cyclische Verbindungen bilden, wobei ein einzelner Ligand mehr als eine Koordinationsstelle an einem Zentralatom besetzt, d. h. mindestens "zweizähnig" ist.

Gebräuchliche und im Rahmen der vorliegenden Erfindung bevorzugte Chelatkomplexbildner sind beispielsweise Polyoxycarbonsäuren, Polyamine, Ethylendiamintetraessigsäure (EDTA), Nitrilotriessigsäure (NTA) und Hydroxyethandiphosphonsäuren bzw. deren Alkalisalze. Auch komplexbildende Polymere, also Polymere, die entweder in der Hauptkette selbst oder seitenständig zu dieser funktionelle Gruppen tragen, die als Liganden wirken können und mit geeigneten Metallatomen in der Regel unter Bildung von Chelat-Komplexen reagieren, sind erfindungsgemäß einsetzbar. Die Polymer-gebundenen Liganden der entstehenden Metall-Komplexe können dabei aus nur einem Makromolekül stammen oder aber zu verschiedenen Polymerketten gehören. Erfindungsgemäß bevorzugte Komplexbildner sind stickstoffhaltigen Polycarbonsäuren, insbesondere EDTA, und Phosphonate, vorzugsweise Hydroxyalkan- bzw. Aminoalkanphosphonate und insbesondere 1-Hydroxyethan-1,1-diphosphonat (HEDP) bzw. dessen Di-oderTetranatriumsalz und/ oder Ethylendiamintetramethylenphosphonat (EDTMP) bzw. dessen Hexanatriumsalz und/oder Diethylentriaminpentamethylenphosphonat (DTPMP) bzw. dessen Hepta- oder Octanatriumsalz.

Weitere, erfindungsgemäß einsetzbare Wirk-, Hilfs- und Zusatzstoffe sind beispielsweise
- nichtionische Polymere wie beispielsweise Vinylpyrrolidinon/Vinylacrylat-Copolymere, Polyvinylpyrrolidinon und Vinylpyrrolidinon/Vinylacetat-Copolymere und Polysiloxane,
- zwitterionische und amphotere Polymere wie beispielsweise Acrylamidopropyltrimethyl-ammoniumchlorid/Acrylat-Copolymere und Octylacrylamid/Methyl-methacrylat/tert-Butylaminoethylmethacrylat/2-Hydroxypropylmethacrylat-Copolymere,
- anionische Polymere wie beispielsweise Polyacrylsäuren, vernetzte Polyacrylsäuren, Vinylacetat / Crotonsäure-Copolymere, Vinylpyrrolidinon/Vinylacrylat-Copolymere, Vinylacetat / Butylmaleat / Isobornylacrylat-Copolymere, Methylvinylether/Malein-säureanhydrid-Copolymere und Acrylsäure/Ethylacrylat/N-tert.Butyl-acrylamid-Terpolymere,
- Verdickungsmittel wie Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie Bentonit oder vollsynthetische Hydrokolloide wie z.B. Polyvinylalkohol,
- Strukturanden wie Zucker, Maleinsäure und Milchsäure und Konsistenzgeber wie Zuckerester, Polyolester oder Polyolalkylether,
- Proteinhydrolysate, insbesondere Elastin-, Kollagen-, Keratin-, Milcheiweiß-, Sojaprotein- und Weizenproteinhydrolysate, deren Kondensationsprodukte mit Fettsäuren,
- Parfümöle,
- Cyclodextrine,
- Lösungsmittel und -vermittler wie Ethanol, Isopropanol, Ethylenglykol, Propylenglykol, Glycerin, Dimethylisosorbid und Diethylenglykol,
- Entschäumer wie Silicone,
- Farbstoffe und Pigmente zum Anfärben des Mittels,
- Antischuppenwirkstoffe wie Piroctone Olamine, Zink Omadine und Climbazol,
- Lichtschutzmittel, insbesondere derivatisierte Benzophenone, Zimtsäure-Derivate und Triazine,
- Wirkstoffe wie Allantoin, Pyrrolidoncarbonsäuren, Cholesterin und deren Salze,
- weitere Fette und Wachse wie Bienenwachs, Montanwachs und Paraffine,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate,
- Trübungsmittel wie Latex, Styrol/PVP- und Styrol/Acrylamid-Copolymere
- Perlglanzmittel wie Ethylenglykolmono- und -distearat sowie PEG-3-distearat,
- Stabilisierungsmittel für Wasserstoffperoxid und andere Oxidationsmittel,
- Treibmittel wie Propan-Butan-Gemische, N₂O Dimethylether, CO₂ und Luft,
- Antioxidantien.

Die Auswahl dieser weiteren Stoffe wird der Fachmann gemäß der gewünschten Eigenschaften der Mittel treffen. Bezüglich weiterer fakultativer Komponenten sowie der eingesetzten Mengen dieser Komponenten wird ausdrücklich auf die dem Fachmann bekannten einschlägigen Handbücher, z. B. Kh. Schrader, Grundlagen und Rezepturen der Kosmetika, 2. Auflage, Hüthig Buch Verlag, Heidelberg, 1989, verwiesen.

Die gebrauchsfertigen Mittel aus zweiphasigem Mittel (M1) und Farbveränderungsmittel (M2) weisen bevorzugt einen pH-Wert im Bereich von 6 bis 12 auf. Erfindungsgemäß bevorzugte Mittel sind dadurch gekennzeichnet, dass sie einen alkalischen pH-Wert besitzen. Eine weitere bevorzugte Ausführungsform der vorliegenden Erfindung besteht darin, dass das anwendungsbereite Mittel einen pH-Wert zwischen 7,0 und 12,0, bevorzugt zwischen 8,0 und 11,0 besitzt. Bei den pH-Werten im Sinne der vorliegenden Erfindung handelt es sich um pH-Werte, die bei einer Temperatur von 22 °C gemessen wurden.

Üblicherweise wird der pH-Wert mit pH-Stellmitteln eingestellt. Zur Einstellung des pH-Werts sind dem Fachmann in der Kosmetik gängige Acidifizierungs- und Alkalisierungsmittel geläufig. Die zur Einstellung des pH-Wertes verwendbaren Alkalisierungsmittel werden typischerweise gewählt aus anorganischen Salzen, insbesondere der Alkali- und Erdalkalimetalle, organischen Alkalisierungsmitteln, insbesondere Aminen, basische Aminosäuren und Alkanolaminen, und Ammoniak. Erfindungsgemäß bevorzugte Acidifizierungsmittel sind Genuss-Säuren, wie beispielsweise Zitronensäure, Essigsäure, Äpfelsäure oder Weinsäure, sowie verdünnte Mineralsäuren.

Erfindungsgemäß einsetzbare, organische Alkalisierungsmittel werden bevorzugt ausgewählt aus Alkanolaminen aus primären, sekundären oder tertiären Aminen mit einem C₂-C₆-Alkylgrund-körper, der mindestens eine Hydroxylgruppe trägt. Erfindungsgemäß ganz besonders bevorzugte Alkanolamine werden ausgewählt aus der Gruppe 2-Aminoethan-1-ol (Monoethanolamin), 2-Amino-2-methylpropan-1-ol und 2-Amino-2-methyl-propan-1,3-diol. Ein insbesondere bevorzugtes Alkanolamin ist Monoethanolamin. Geeignete basische Aminsäuren sind Lysin, Arginin und Ornithin. Das erfindungsgemäße, anorganische Alkalisierungsmittel sind bevorzugt ausgewählt aus der Gruppe, die gebildet wird aus Natriumhydroxid, Kaliumhydroxid, Calciumhydroxid, Bariumhydroxid, Natriumphosphat, Kaliumphosphat, Natriumsilicat, Kaliumsilicat, Natriumcarbonat und Kaliumcarbonat.

Die erfindungsgemäßen Zusammensetzungen enthalten die Alkalisierungsmittel bevorzugt in Mengen von 0,2 bis 25 Gew.-%, insbesondere 0,5 bis 10 Gew.-%.

Die Anwendungstemperaturen können in einem Bereich zwischen 15 und 40 °C liegen. Nach einer Einwirkungszeit von 2 bis 60, bevorzugt 5 bis 45 Minuten wird das Blondiermittel durch Ausspülen von dem Haar entfernt. Das Nachwaschen mit einem Shampoo entfällt, wenn ein stark tensidhaltiger Träger verwendet wurde.

Je nach Zusammensetzung der Mittel (M1) und (M2) kann erfindungsgemäß bevorzugt sein, die Mittel erst unmittelbar vor der Anwendung durch Vermischen von Mittel (M1) und Mittel (M2) herzustellen. Dies ist insbesondere bei Inkompatibilitäten zwischen einzelnen Inhaltsstoffen vorteilhaft sein. Daher ist eine bevorzugte Darreichungsform des anwendungsbereiten Mittels eine getrennte Verpackungseinheit, worin die Mittel (M1) und (M2) jeweils getrennt voneinander verpackt vorliegen.

Ein weiterer Gegenstand der vorliegenden Erfindung ist daher eine Verpackungseinheit (Kit-of-Parts), welche mindestens zwei voneinander getrennt konfektionierten Containern enthält, wobei ein erster Container (C1) ein kosmetisches Mittel (M1) gemäß dem ersten Erfindungsgegenstand enthält und ein zweiter Container (C2) eine Farbveränderungszubereitung (M2), enthaltend in einem kosmetischen Träger mindestens eine farbverändernde Komponente, enthält.

Unter Container wird im Rahmen der vorliegenden Erfindung eine Umhüllung verstanden, die in Form einer gegebenenfalls wieder-verschließbaren Flasche, einer Tube, einer Dose, eines Tütchens, eines Sachets oder ähnlichen Umhüllungen vorliegt. Dem Umhüllungsmaterial sind erfindungsgemäß keine Grenzen gesetzt. Bevorzugt handelt es sich jedoch dabei um Umhüllungen aus Glas oder Kunststoff. Besonders bevorzugt zur Visualisierung der zweiphasigen Mittel (M1) ist eine Ausführungsform, bei der die Umhüllung des Containers, welcher das Mittel (M1) enthält, für den Anwender durchsichtig ist. Eine bevorzugte Ausführungsform der erfindungsgemäßen Mehrkomponentenverpackungseinheit ist daher dadurch gekennzeichnet, dass der erste Container (C1), enthaltend das Mittel (M1), eine durchsichtige Verpackung, bevorzugt eine durchsichtige Kunststoffverpackung, besitzt.

Eine weitere Ausführungsform dieses Erfindungsgegenstands ist gegeben, wenn das Mittel (M2) aus Container (C2) die Färbezubereitung darstellt und als farbverändernde Komponente mindestens ein Oxidationsfarbstoffvorprodukt und/oder mindestens einen direktziehenden Farbstoff und/oder mindestens ein Aufhellmittel enthält.

Weiterhin kann es erfindungsgemäß besonders vorteilhaft sein, wenn das genannte Kit-of-Parts mindestens ein weiteres Haarbehandlungsmittel in einem getrennten Container enthält, insbesondere ein Konditioniermittel. Darüber hinaus kann die Verpackungseinheit Applikationshilfen, wie Kämme, Bürsten oder Pinsel, persönliche Schutzkleidung, insbesondere Ein-Weg-Handschuhe, sowie gegebenenfalls eine Gebrauchsanleitung umfassen.

Hinsichtlich der bevorzugten Ausführungsführungsformen der Mittel (M1) und (M2) gelten mutatis mutandis die obigen Ausführungen der vorangehenden Erfindungsgegenstände.

Bei der Anwendung der Mehrkomponentenverpackungseinheit kann es unerheblich sein, ob zunächst die beiden Phasen von Mittel (M1) durch kräftiges Schütteln kurzzeitig durchmischt werden und vor erneuter Phasentrennung das Mittel (M2) zugegeben wird, um die anwendungsbereite Farbveränderungszubereitung bereitzustellen, oder ob zunächst das Mittel (M2) zum Mittel (M1) gegeben wird und anschließend durch inniges Vermischen die anwendungsbereite Mischung hergestellt wird.

Zur verbesserten Durchmischung ist vorteilhaft, wenn der Container (C1), welcher das zweiphasige Mittel (M1) enthält, eine wieder-verschließbare Öffnung, wie beispielsweise einen Schnapp- oder einen Schraubverschluss, besitzt. Dies ermöglicht die erleichterte Zugabe des farbverändernden Mittels aus Container (C2), welcher seinerseits bevorzugt in Form eines Tütchens oder Sachets im Falle von wasserfreien, insbesondere pulverförmigen Farbveränderungsmitteln, oder in Form einer Tube im Falle von fließfähigen Farbveränderungsmitteln.

Es ist bevorzugt, die einzelnen Zubereitungen zu vermischen und das anwendungsbereite Mittel zeitnah auf die keratinischen Fasern zu applizieren.

Ein weiterer Erfindungsgegenstand ist daher Verfahren zur Farbveränderung keratinischer Fasern, insbesondere menschlicher Haare, welches dadurch gekennzeichnet ist, dass aus einer Mehrkomponentenverpackungseinheit gemäß dem vorangegangenen Erfindungsgegenstand der Inhalt des Containers (C2) in den Container (C1) gefüllt wird, der wiederverschlossene Container (C1) daraufhin geschüttelt wird, und das im Container (C1) resultierende, anwendungsbereite Farbveränderungsmittel anschließend auf die Fasern aufgebracht wird, für eine Einwirkdauer von 5 bis 60 min auf den Fasern belassen und schließlich ausgespült wird.

Im Falle eines farbgebenden Mittels beträgt die bevorzugte Einwirkzeit 5 bis 40 min, bevorzugt 10 bis 30 min. Im Falle von aufhellenden oder bleichenden Farbveränderungsmitteln liegt die bevorzugte Einwirkzeit bei 30 bis 60 min, bevorzugt bei 40 bis 60 min.

Ein weiterer Erfindungsgegenstand ist schließlich ein Verfahren zur Farbveränderung keratinischer Fasern, insbesondere menschlicher Haare, welches dadurch gekennzeichnet ist, dass aus einer Mehrkomponentenverpackungseinheit gemäß dem vorangegangenen Erfindungsgegenstand der Container (C1) geschüttelt wird, die entstandene Mischung der Phasen (I) und (II) unmittelbar anschließend mit einer Färbezubereitung des Containers (C2) innig vermischt, das resultierende, anwendungsbereite Farbveränderungsmittel anschließend auf die Fasern aufgebracht wird, für eine Einwirkdauer von 5 bis 60 min auf den Fasern belassen und schließlich ausgespült wird.

Im Rahmen dieses Gegenstandes der Erfindung gelten mutatis mutandis die oben getroffenen Aussagen analog.

Die folgenden Beispiele sollen den Gegenstand der vorliegenden Erfindung näher erläutern ohne diesen in irgendeiner Weise zu beschränken.

### Beispiele

**1. Rezepturen**

| **Rohstoffe** | **Gew.-%** |
|---|---|
| Gluadin WQ | 1,00 |
| Na-benzoat | 0,10 |
| Glycin | 0,20 |
| Puricare LS 9658 | 0,10 |
| D-Panthenol 75 % | 0,50 |
| Luviquat FC 550 | 1,00 |
| Genamin CTAC | 0,50 |
| Milchsäure | 0,66 |
| Dow Corning 5225 C Formulation Aid | 3,50 |
| Dow Corning 556 | 1,00 |
| Dow Corning DB 1411 | 3,50 |
| Parsol SLX | 1,00 |
| Dipicolinsäure | 0,10 |
| Dinatriumpyrophosphat | 0,03 |
| Turpinal SL | 1,50 |
| Dow Corning DB 110 A | 0,07 |
| Aculyn 33A | 12,00 |
| Wasserstoffperoxid 50 % | 22,40 |
| Parfum | qs |
| Wasser | ad 100 |

Gluadin WQ (ca. 31% Aktivsubstanzgehalt; INCI-Bezeichnung: Laurimonium Hydroxypropyl Hydrolyzed Wheat Protein; Cognis); Puricare LS 9658 (ca. 0.1-1 % Aktivsubstanzgehalt; INCI-Bezeichnung: Aqua, Glycerine, Moringa Pterygosperma Seed Extract; Laboratoires Serobiologiques); Luviquat FC 550 (ca. 40% Aktivsubstanzgehalt; INCI-Bezeichnung: Polyquaternium-16; BASF); Genamin CTAC (ca. 30% Aktivsubstanzgehalt; INCI-Bezeichnung: Cetrimonium Chloride; Clariant); Dow Corning 5225 C (INCI-Bezeichnung: Cyclomethicone, PEG/PPG-18/18 Dimethicone; Dow Corning); Dow Corning 556 (INCI-Bezeichnung: Phenyl Trimethicone; Dow Corning); Dow Corning DB 1411 (INCI-Bezeichnung: Cyclomethicone, Dimethicone; Dow Corning); Parsol SLX (INCI-Bezeichnung: Polysilicone-15; DSM); Turpinal SL (ca. 60% Aktivsubstanzgehalt; INCI-Bezeichnung: Etidronic Acid, Aqua; Solutia); Aculyn 33 (ca. 28% Festkörper in Wasser; INCI-Bezeichnung: Acrylates Copolymer; Rohm & Haas); Dow Corning DB 110 A (INCI-Bezeichnung: Dimethicon; Dow Corning).

## Patentansprüche

1. Kosmetisches Mittel für die Behandlung keratinischer Fasern, welches mindestens zwei voneinander getrennte Phasen umfasst,
wobei die erste Phase (I) eine wässrige Phase darstellt, die mindestens ein chemisches Oxidationsmittel enthält, und
wobei die zweite Phase (II) eine hydrophobe Phase darstellt, die mindestens ein Siliconöl enthält,
**dadurch gekennzeichnet,**
**dass** die beiden Phasen in zwei Schichten übereinander vorliegen,
**dass** das Mittel nichtionische, anionische, zwitterionische und/oder amphotere Tenside und/oder Emulgatoren in einem Gesamtgewicht von weniger als 5 Gew.-%, bezogen auf das Gesamtgewicht des Mittels, enthält,
**dass** das Mittel als hydrophobe Phase (II) mindestens ein Siliconöl, ausgewählt aus der Gruppe, die gebildet wird aus Dimethiconen und Cyclomethiconen, enthält und
**dass** das Mittel zusätzlich mindestens eine überwiegend im Siliconöl lösliche Pflegekomponente, ausgewählt aus öllöslichen Pflegestoffen, öllöslichen Vitaminen und Triglyceriden, enthält.

2. Mittel nach Anspruch 1, **dadurch gekennzeichnet, dass** das chemische Oxidationsmittel der Phase (I) ausgewählt ist aus Wasserstoffperoxid und/oder einem seiner festen Anlagerungsprodukte an anorganische und/oder organische Verbindungen.

3. Mittel nach einem der Ansprüche 1 bis 2, **dadurch gekennzeichnet, dass** das Mittel nicht-ionische, anionische, zwitterionische und/oder amphotere Tenside und/oder Emulgatoren in einem Gesamtgewicht von weniger als 1 Gew.-%, bezogen auf das Gesamtgewicht des Mittels, enthält.

4. Mehrkomponentenverpackungseinheit (Kit-of-Parts), enthaltend mindestens zwei voneinander getrennt konfektionierte Container, wobei ein erster Container (C1) ein kosmetisches Mittel gemäß einem der Ansprüche 1 bis 3 enthält und ein zweiter Container (C2) eine Farbveränderungszubereitung, enthaltend in einem kosmetischen Träger mindestens eine farbverändernde Komponente, enthält.

5. Mehrkomponentenverpackungseinheit gemäß Anspruch 4, **dadurch gekennzeichnet, dass** der erste Container (C1) eine durchsichtige Verpackung, bevorzugt eine durchsichtige Kunststoffverpackung, besitzt.

6. Mehrkomponentenverpackungseinheit gemäß einem der Ansprüche 4 oder 5, **dadurch gekennzeichnet, dass** die Farbveränderungszubereitung als farbverändernde Komponente mindestens ein Oxidationsfarbstoffvorprodukt und/oder mindestens einen direktziehenden Farbstoff und/oder mindestens ein Aufhellmittel enthält.

7. Verfahren zur Farbveränderung keratinischer Fasern, insbesondere menschlicher Haare, **dadurch gekennzeichnet, dass** aus einer Mehrkomponentenverpackungseinheit gemäß einem der Ansprüche 4 bis 6 der Inhalt des Containers (C2) in den Container (C1) gefüllt wird, der wiederverschlossene Container (C1) daraufhin geschüttelt wird, und das im Container (C1) resultierende, anwendungsbereite Farbveränderungsmittel anschließend auf die Fasern aufgebracht wird, für eine Einwirkdauer von 5 bis 60 min auf den Fasern belassen und schließlich ausgespült wird.

8. Verfahren zur Farbveränderung keratinischer Fasern, insbesondere menschlicher Haare, **dadurch gekennzeichnet, dass** aus einer Mehrkomponentenverpackungseinheit gemäß einem der Ansprüche 4 bis 6 der Container (C1) geschüttelt wird, die entstandene Mischung der Phasen (I) und (II) unmittelbar anschließend mit einer Färbezubereitung des Containers (C2) innig vermischt, das resultierende, anwendungsbereite Farbveränderungsmittel anschließend auf die Fasern aufgebracht wird, für eine Einwirkdauer von 5 bis 60 min auf den Fasern belassen und schließlich ausgespült wird.

## Claims

1. A cosmetic agent for treating keratinic fibers, which comprises at least two phases that are separate from one another,
the first phase (I) being an aqueous phase which contains at least one chemical oxidizing agent, and
the second phase (II) being a hydrophobic phase which contains at least one silicone oil, **characterized in that**,
the two phases are in two layers one on top of the other,
**in that** the agent contains non-ionic, anionic, zwitterionic and/or amphoteric surfactants and/or emulsifiers in a total weight of less than 5 wt. % based on the total weight of the agent, **in that** the agent contains, as the hydrophobic phase (II), at least one silicone oil selected from the group formed of dimethicones and cyclomethicones, and
**in that** the agent contains in addition at least one nourishing component that can be predominantly dissolved in silicone oil and is selected from oil-soluble nourishing ingredients, oil-soluble vitamins and triglycerides.

2. The agent according to claim 1, **characterized in that** the chemical oxidizing agent of phase (I) is selected from hydrogen peroxide and/or one of its solid addition products with inorganic and/or organic compounds

3. The agent according to one of claims 1 to 2, **characterized in that** the agent contains nonionic, anionic, zwitterionic and/or amphoteric surfactants and/or emulsifiers in a total weight of less than 1 wt.%, based on the total weight of the agent.

4. A kit of parts, containing at least two separately packaged containers, wherein a first container (C1) contains a cosmetic agent according to one of claims 1 to 3 and a second container (C2) contains a color-changing preparation containing at least one color-changing component in a cosmetic carrier.

5. The kit of parts according to claim 4, **characterized in that** the first container (C1) has a transparent packaging, preferably a transparent plastics packaging.

6. The kit of parts according to one of claims 4 or 5, **characterized in that** the color-changing preparation contains at least one oxidation dye precursor and/or at least one direct dye and/or at least one lightening agent as the color-changing component.

7. A method for changing the color of keratinic fibers, in particular human hair, **characterized in that** the contents of the container (C2) is put into the container (C1) from a kit of parts according to one of claims 4 to 6, the container (C1) is closed again and then shaken, and the resulting ready-to-use color-changing agent in the container (C1) is then applied to the fibers, left on the fibres for a reaction time of from 5 to 60 minutes and then rinsed out.

8. The method for changing the color of keratinic fibres, in particular human hair, **characterized in that** the container (C1) is shaken from a kit of parts according to one of claims 4 to 6, the resulting mixture of phases (I) and (II) is then directly intimately mixed with a color preparation of the container (C2), the resulting ready-to-use color-changing agent is then applied to the fibres, left on the fibres for a reaction time of from 5 to 60 minutes and then rinsed out.

## Revendications

1. Agent cosmétique pour le traitement de fibres de kératine qui comprend au moins deux phases distinctes,
la première phase (I) étant une phase aqueuse qui contient au moins un agent d'oxydation chimique, est
la deuxième phase (II) étant une phase hydrophobe qui contient au moins une huile de silicone,
**caractérisé en ce que**
les deux phases sont présentes en deux couches l'une au-dessus de l'autre,
l'agent contient des tensioactifs non ioniques, anioniques, zwitterioniques et/ou amphotères et/ou des émulsifiants dans un poids total de moins de 5% poids par rapport au poids total de l'agent,
l'agent contient comme phase hydrophobe (II) au moins une huile de silicone choisie dans le groupe qui est formé par les diméthicones et les cyclométhicones et
l'agent contient en outre au moins un composant de soin soluble essentiellement dans l'huile de silicone, choisi parmi les substances de soins solubles dans l'huile, les vitamines solubles dans l'huile et les triglycérides.

2. Agent selon la revendication 1, **caractérisé en ce que** l'agent d'oxydation chimique de la phase (I) est choisi parmi le peroxyde d'hydrogène et/ou ses produits d'addition solides à des composés minéraux et/ou organiques.

3. Agent selon l'une des revendications 1 à 2, **caractérisé en ce que** l'agent contient des tensioactifs non ioniques, unioniques, zwitterioniques et/ou amphotères et/ou des émulsifiants d'un poids total de mains de 1% en poids, par rapport au poids total de l'agent.

4. Unité d'emballage multi-composant (kit-of-parts) comprenant au moins deux récipients confectionnés séparément l'un de l'autre, un premier récipient (C1) contenant un produit cosmétique selon l'un des revendications 1 à 3 et un second récipient (C2) contenant une composition de changement de couleur qui contient dans un support cosmétique au moins un composant de changement de couleur.

5. Unité d'emballage multi-composant selon la revendication 4, **caractérisée en ce que** le premier récipient (C1) possède un emballage transparent, de préférence un emballage en matière plastique transparente.

6. Unité d'emballage multi-composant selon l'une des revendications 4 ou 5, **caractérisée en ce que** la composition de changement de couleur contient comme composant de
changement de couleur au moins un précurseur de colorant d'oxydation et/ou au moins un colorant direct et/ou au moins un azurant.

7. Procédé de changement de couleur de fibres de kératine, en particulier de cheveux humains, **caractérisé en ce que**, à partir d'une unité d'emballage multi-composant selon l'une quelconque des revendications 4 à 6, on introduit le contenu du récipient (C2) dans le récipient (C1), on agite ensuite le récipient (C1) refermé puis on applique l'agent de changement de couleur résultant prêt à l'emploi, qui se trouve dans le récipient (C1), sur les fibres, on le laisse sur les fibres pendant une période d'action de 5 à 60 minutes, et enfin on le rince.

8. Procédé pour changer la couleur de fibres de kératine, en particulier de cheveux humains, **caractérisé en ce que**, à partir d'une unité d'emballage multi-composant selon l'une quelconque des revendications 4 à 6, on agite le conteneur (C1), on mélange intimement, immédiatement après, le mélange résultant des phases (I) et (II) avec une composition colorante du récipient (C2) puis on applique l'agent de changement de couleur résultant prêt à l'emploi sur les fibres, ensuite on le laisse sur les fibres pendant une période d'action de 5 à 60 minutes, et enfin on le rince
